# FASCICULE DE BREVET EUROPEEN

(11) **EP 1 441 530 B1**
(45) Date de publication et mention de la délivrance du brevet: **28.04.2010**
(21) Numéro de dépôt: 04290014.2
(22) Date de dépôt: 06.01.2004
(51) Int. Cl.: H04N 7/18, A61B 1/05

(54) **Vidéoendoscope**
Videoendoskop
Video endoscope

(30) Priorité: 17.01.2003 FR 0300547; 28.01.2003 FR 0300916; 31.01.2003 FR 0301134; 22.05.2003 FR 0306180
(43) Date de publication de la demande: 28.07.2004
(73) Titulaire: Tokendo, 13600 La Ciotat (FR)
(72) Inventeur: Rovegno, Jean, 13600 La Ciotat (FR)
(74) Mandataire: de Roquemaurel, Bruno

(56) Documents cités:
- FR-A- 2 785 132
- US-A- 5 149 446
- US-A- 5 373 317
- US-A- 6 126 591
- US-A1- 2002 032 365
- PATENT ABSTRACTS OF JAPAN vol. 1999, no. 14, 22 décembre 1999 (1999-12-22) -& JP 11 244225 A (OLYMPUS OPTICAL CO LTD), 14 septembre 1999 (1999-09-14)
- PATENT ABSTRACTS OF JAPAN vol. 2000, no. 21, 3 août 2001 (2001-08-03) -& JP 2001 095754 A (OLYMPUS OPTICAL CO LTD), 10 avril 2001 (2001-04-10)

## Description

La présente invention concerne un vidéoendoscope destiné notamment, mais non exclusivement à l'endoscopie à vocation industrielle.

On désigne généralement par le terme "endoscope" une sonde souple ou rigide, destinée à être introduite dans une cavité obscure et permettant à son utilisateur d'observer au travers d'un oculaire une cible située dans la cavité. A cet effet, un endoscope intègre un dispositif d'illumination de la cible et un dispositif optique fournissant à l'utilisateur une image optique de la cible. Le dispositif optique comprend un objectif distal, un dispositif de transport d'images rigide constitué d'une série de lentilles, ou souple constitué d'un faisceau de fibres optiques ordonnées, et un oculaire proximal au travers duquel l'utilisateur peut observer visuellement l'image de la cible. Le dispositif d'illumination est généralement constitué d'un faisceau de fibres d'éclairage dont l'extrémité distale, convenablement disposée à proximité de l'objectif distal, illumine la cible quand son extrémité proximale est connectée à un générateur de lumière.

On désigne par le terme "vidéoendoscope" une sonde souple ou rigide permettant à son utilisateur d'observer sur un écran vidéo l'image d'une cible située dans une cavité obscure. A cet effet, un vidéoendoscope comprend un généralement les éléments suivants :
- un embout distal logeant un dispositif optoélectronique comportant notamment un objectif et un capteur CCD (Charge Coupled Device) sur la surface sensible duquel se forme l'image de la cible délivrée par l'objectif,
- un tube d'inspection souple dont l'extrémité distale est solidaire de l'embout distal,
- une poignée de commande solidaire de l'extrémité proximale du tube d'inspection,
- un câble ombilical souple de raccordement dont l'extrémité distale est solidaire de la poignée de commande et dont l'extrémité proximale est destinée à être raccordée à un coffret externe intégrant un générateur de lumière et une source d'alimentation électrique,
- un faisceau de fibres d'éclairage logé dans le câble ombilical, dans la poignée de commande, puis dans le tube d'inspection et dont l'extrémité distale, logée dans l'embout distal, illumine la cible lorsque son extrémité proximale est connectée à un générateur de lumière,
- un processeur vidéo relié électriquement au capteur CCD, et dont la synchronisation est réglée en fonction de la longueur du câble électrique le reliant au capteur CCD,
- un écran vidéo relié au processeur vidéo, et
- un panneau de commande permettant de régler le fonctionnement du processeur vidéo et éventuellement de l'écran vidéo.

Les vidéoendoscopes souples disposent en outre d'un béquillage distal articulé permettant de modifier l'orientation de l'embout distal de la sonde, la poignée de commande intégrant alors généralement des moyens de commande mécaniques ou électromécaniques permettant d'actionner ce béquillage. Les moyens de commande électromécaniques du béquillage distal comportent généralement deux moteurs logés dans la poignée de commande, actionnant des poulies respectives reliées par des câbles au béquillage distal, ces moteurs étant commandés à l'aide d'un manche à balai intégré dans le panneau de commande.

Les vidéoendoscopes de conception récente peuvent en outre être associés à un système numérique de gel, de traitement et d'enregistrement d'images susceptible d'être directement géré par le panneau de commande du vidéoendoscope.

Dans les architectures retenues par les divers constructeurs de vidéoendoscopes actuels :
- le processeur vidéo est intégré soit dans la poignée de commande, soit dans le boîtier de connexion de la sonde, soit dans le coffret externe renfermant le générateur de lumière,
- le moniteur vidéo peut être intégré à la poignée de commande, ou se connecter au coffret externe,
- le panneau de commande du processeur vidéo et du moniteur vidéo peut être intégré soit dans la poignée de commande, soit dans le coffret externe.

La poignée de commande de vidéoendoscope faisant l'objet de la présente invention regroupe le moniteur vidéo, le panneau de commande du processeur vidéo, et éventuellement les commandes du béquillage, le processeur vidéo étant logé soit dans un boîtier de connexion constituant l'extrémité proximale du câble ombilical, soit de façon préférentielle dans la poignée de commande. Habituellement, les poignées de commande de ce type se présentent sous la forme d'un boîtier de forme allongée, solidaire d'un moniteur vidéo disposé transversalement sur ledit boîtier. Le tube d'inspection est solidaire de l'extrémité distale du boîtier, tandis que le câble ombilical est solidaire de son extrémité proximale. Généralement, le panneau de commande est intégré dans le boîtier de commande.

Il en résulte que les dimensions du moniteur vidéo sont nécessairement réduites afin de ne pas alourdir la poignée et déséquilibrer son centre de gravité. Il est alors souvent nécessaire de connecter le vidéoendoscope à un moniteur vidéo externe de plus grandes dimensions, ce qui affecte directement l'autonomie et la mobilité de l'équipement.

En outre, l'utilisateur est gêné par le câble ombilical solidaire de l'extrémité proximale de la poignée de commande.

Une telle poignée de commande a été décrite dans la demande de brevet FR 2 785 132 déposée par la Demanderesse et dans le brevet US 5 373 317.

Le brevet US 4 539 586 décrit un module de connexion d'une sonde vidéoendoscopique permettant de raccorder la sonde à un coffret externe adapté spécialement, intégrant notamment un générateur de lumière, un processeur vidéo et une alimentation électrique. Ce module de connexion comprend des circuits électroniques tels que des amplificateurs et des compensateurs de retard d'horloge, qui sont réglés en fonction de la longueur de la sonde, de manière à permettre de connecter des sondes de longueurs différentes.

La demande de brevet EP 0 587 514 décrit également un module de connexion de sonde vidéoendoscopique, intégrant un processeur vidéo réglé en fonction de la longueur de la sonde, ce module de connexion étant enfichable dans un coffret externe spécifique, logeant un générateur de lumière et une alimentation électrique.

Les vidéoendoscopes décrits dans ces documents imposent à l'utilisateur l'acquisition du coffret externe spécifiquement adapté, intégrant un générateur de lumière et une alimentation électrique, présentant des caractéristiques également spécifiques. Or il est fréquent que les utilisateurs de telles sondes vidéoendoscopiques disposent déjà de générateurs de lumière pour de telles sondes. En outre, il est préférable de pouvoir disposer de plusieurs générateurs de lumière pour pouvoir continuer à utiliser la sonde même en cas de panne de l'un d'entre eux.

Un exemple de dispositif électromécanique de commande du béquillage distal est par exemple décrit dans le brevet US 4 941 454. Ce dispositif électromécanique comprend deux servomoteurs actionnant chacun une poulie entraînant une paire de câbles dont les deux extrémités sont fixées au béquillage distal pour transmettre au béquillage distal les mouvements de la poulie et ainsi modifier l'orientation du béquillage distal articulé dans un plan, les deux plans dans lesquels l'orientation du béquillage distal est modifiée respectivement par les deux servomoteurs étant perpendiculaires l'un à l'autre.
Ce dispositif comporte également un circuit de commande des servomoteurs générant pour chaque servomoteur un train d'impulsions dont la largeur est ajustée à l'aide d'un potentiomètre. Les deux potentiomètres sont actionnés mécaniquement par les déplacements dans deux plans perpendiculaires d'un manche à balai manuellement actionné par l'utilisateur.

En raison de l'usage d'un manche à balai actionnant des potentiomètres, une action manuelle prolongée sur le manche à balai entraîne une déviation permanente du manche et donc une orientation spécifique permanente du béquillage distal. Par contre, dès que l'utilisateur relâche le manche à balai, ce dernier revient dans sa position neutre. Il en est de même du béquillage.

De plus, il s'avère que cette solution ne permet pas de déterminer la position angulaire des poulies. Cet inconvénient est particulièrement gênant étant donné que l'utilisateur ne peut appréhender naturellement l'orientation du béquillage dont les mouvements sont commandés par un manche à balai.

En principe, lorsque le manche à balai est dans sa position médiane le béquillage présente une orientation neutre, c'est-à-dire se trouve dans l'axe de l'extrémité distale de la sonde. Par conséquent, si le manche à balai présente une position déviée permanente, le béquillage présente également une orientation déviée permanente, ce qui rend un tel dispositif peu commode à utiliser.
Le brevet US 5 658 238 décrit un autre exemple de dispositif électromécanique de commande de béquillage distal comportant des moteurs à courant continu commandés par des boutons de commande, l'électronique de commande de ce dispositif étant disposée dans un boîtier externe. Ce dispositif nécessite de prévoir des encodeurs pour déterminer les positions angulaires respectives des moteurs et donc une électronique d'asservissement de ces positions angulaires.
Les endoscopes et vidéoendoscopes présentant un tube d'inspection souple sont généralement rangés à l'aide d'un tambour autour duquel le tube d'inspection est enroulé.

Le brevet US 4 913 369 décrit un tel tambour monté rotatif sur une base et comprenant un moyeu en mousse présentant des logements prévus pour ranger l'embout distal de la sonde et le connecteur situé à l'extrémité proximale, ces logements étant refermés par un couvercle latéral. Il en résulte qu'un tel tambour doit être préalablement retiré de son coffret de rangement pour pouvoir utiliser la sonde.

Le brevet EP 0 276 139 et la demande de brevet US 2002/0 032 365 décrivent des tambours de rangement d'une sonde vidéoendoscopique autour desquels la sonde est enroulée en commençant par son extrémité distale, le moyeu du tambour intégrant des moyens de connexion de l'extrémité proximale de la sonde à une source électrique et une source de lumière. De cette manière, la sonde peut être utilisée sans avoir à la dérouler complètement du tambour. Toutefois, cette solution présente l'inconvénient de nécessiter d'intégrer la source de lumière et le processeur vidéo dans le moyeu du tambour et de prévoir des connecteurs électriques tournants pour alimenter la sonde, de tels moyens de connexion étant très coûteux, peu fiables à long terme et introduisant des pertes électriques. En outre, cette solution rend la sonde inutilisable avec un autre coffret de rangement

Par ailleurs, le brevet US 5 314 070 décrit un coffret de rangement d'un tube d'inspection d'endoscope ou de vidéoendoscope comportant un tube de rangement enroulé en spirale, dans lequel on engage le tube d'inspection. Il en résulte qu'à chaque insertion et extraction du coffret de rangement, le tube d'inspection et en particulier son embout distal frotte contre la paroi intérieure du tube de rangement. A partir d'une certaine longueur de tube d'inspection engagée dans le tube de rangement, la force nécessaire pour vaincre les forces de frottement devient très importante et risque d'endommager le tube d'inspection et en particulier la jonction entre celui-ci et la poignée de commande, par une torsion exagérée du tube d'inspection. Ce risque de torsion nécessite de renforcer la gaine du tube d'inspection au moins au voisinage du boîtier de commande, ce qui entraîne des coûts supplémentaires. Par ailleurs, cette solution ne convient pas aux endoscopes comportant un tube d'inspection de grande longueur, notamment supérieure à 6 m.

La présente invention a pour but d'améliorer l'ergonomie d'un tel vidéoendoscope. Cet objectif est atteint par la prévision d'un vidéoendoscope comprenant :
- une sonde d'inspection comprenant un tube d'inspection comportant un embout distal logeant un dispositif optoélectronique d'imagerie délivrant un signal électrique,
- un processeur vidéo pour traiter le signal électrique délivré par le dispositif d'imagerie afin de générer un signal vidéo,
- une poignée de commande comprenant un boîtier de commande solidaire de l'extrémité proximale du tube d'inspection et muni de moyens de commande et de réglage du processeur vidéo,
- un câble ombilical de raccordement dont l'extrémité distale est solidaire du boîtier de commande, pour connecter le vidéoendoscope à une source de lumière et à une source d'alimentation électrique,
- un boîtier de visualisation intégrant un écran vidéo d'affichage connecté au processeur vidéo pour visualiser le signal vidéo, et
- un faisceau de fibres d'éclairage intégré sans rupture de continuité dans le câble ombilical, dans la poignée de commande, puis dans le tube d'inspection et dont l'extrémité distale, logée dans l'embout distal, illumine une cible observée par la sonde lorsque l'extrémité proximale du câble ombilical est connectée à un générateur de lumière.

dans lequel, le boîtier de visualisation est solidaire d'une face latérale du boîtier de commande, le boîtier de commande comprenant sur une face supérieure un panneau de commande muni des moyens de commande et de réglage du processeur vidéo, et présentant une forme allongée entre son extrémité distale et son extrémité proximale, de manière à pouvoir être tenu dans une main et à permettre l'actionnement des organes de commande à l'aide du pouce de ladite main, caractérisé en ce que le boîtier de visualisation est solidarisé avec le boîtier de commande par une articulation permettant d'orienter l'écran d'affichage autour d'un axe perpendiculaire à la face latérale du boîtier de commande, le boîtier de visualisation étant couplé électriquement au boîtier de commande par l'intermédiaire de conducteurs électriques passant dans l'articulation.

Avantageusement, le câble ombilical est raccordé au boîtier de commande par une face latérale de celui-ci.

Avantageusement, l'articulation est formée d'un connecteur assurant un raccordement mécanique et électrique amovible du boîtier de visualisation au boîtier de commande.

Selon un mode de réalisation de l'invention, l'extrémité proximale du câble ombilical est solidaire d'un boîtier de connexion équipé de moyens de connexion de l'extrémité proximale du faisceau de fibres d'éclairage à un générateur de lumière, et de moyens de connexion de la sonde vidéoendoscopique à une source d'alimentation électrique.

Avantageusement, l'embout de connexion comprend des moyens de fixation pour fixer un adaptateur mécanique pour adapter l'embout de connexion à l'embout de connexion de n'importe quel générateur de lumière, le panneau de commande comprend un organe de commande pour commander l'initialisation du processeur vidéo en fonction de la température de couleur de la lampe du générateur de lumière.

De préférence, le processeur vidéo est intégré dans le boîtier commande.

Selon un mode de réalisation de l'invention, le boîtier de commande comprend sur ses deux faces latérales des moyens d'accouplement mécanique et électrique pour la fixation et la connexion électrique du boîtier de visualisation indifféremment sur l'une ou l'autre des faces latérales du boîtier de commande.

Alternativement, le processeur vidéo est intégré dans le boîtier de visualisation ou dans un boîtier de connexion de l'extrémité proximale du câble ombilical à un coffret externe.

Subsidiairement, la présente invention a également pour but d'offrir à l'utilisateur la possibilité de raccorder une sonde vidéoendoscopique disposant de son propre processeur vidéo, à un générateur de lumière de son choix, à une alimentation électrique banalisée et/ou à un coffret externe intégrant notamment un système de traitement et de stockage d'images vidéo.
Cet objectif est atteint par le fait que le boîtier de connexion comprend en outre des moyens de connexion de la sonde à un système de traitement et/ou de stockage d'images, la sonde vidéoendoscopique comprenant en outre des moyens de commutation conçus pour orienter vers l'écran vidéo soit le signal vidéo issu du processeur vidéo, soit le signal vidéo issu du système de traitement et/ou de stockage d'images.

Avantageusement, le boîtier de commande comprend des moyens pour commander un système de traitement et/ou de stockage d'images connecté aux moyens de connexion du dispositif de connexion, ces moyens de connexion comprenant une broche pour transmettre le signal vidéo généré par le processeur vidéo à une entrée vidéo du système de traitement et/ou de stockage d'images, une broche pour transmettre au dispositif de commutation un signal vidéo généré par le système de traitement et/ou de stockage d'images, et une broche reliant les moyens de commande du boîtier de commande à une interface de commande du système de traitement et/ou de stockage d'images.

De préférence, le vidéoendoscope comprend en outre des moyens pour commander les moyens de commutation pour orienter automatiquement vers l'écran vidéo le signal vidéo issu du système de traitement et/ou de stockage d'images dès que celui-ci est raccordé aux moyens de connexion.

Selon un mode de réalisation de l'invention, les moyens de commutation sont intégrés dans le boîtier de commande, ou dans le boîtier de visualisation, ou encore dans le dispositif de connexion.

Selon un mode de réalisation de l'invention, les moyens de connexion de la sonde à un système de traitement et/ou de stockage d'images comprennent une broche de connexion permettant d'alimenter le vidéoendoscope à partir d'une alimentation électrique associée au système de traitement et/ou de stockage d'images.

Selon un mode de réalisation de l'invention, le dispositif de connexion comprend des moyens de connexion de la sonde vidéoendoscopique à une source d'alimentation électrique auxiliaire.

Selon un mode de réalisation de l'invention, le dispositif de connexion comprend des moyens de connexion de la sonde vidéoendoscopique à un moniteur vidéo auxiliaire, les moyens de commutation comportant des moyens pour envoyer le signal vidéo appliqué en entrée de l'écran vidéo, vers les moyens de connexion d'un moniteur vidéo auxiliaire.

Avantageusement, la poignée de commande comprend des moyens pour commander les moyens de commutation.

Subsidiairement, la présente invention a également pour but d'équiper un tel vidéoendoscope d'un béquillage distal permettant de modifier l'orientation de l'embout distal de la sonde pour modifier le champ de vision, tout en supprimant les inconvénients des solutions de l'art antérieur précédemment décrit. Cet objectif est atteint par le fait que le boîtier de commande intègre en outre :
- un dispositif électromécanique conçu pour déformer un béquillage distal déformable, intégré à l'extrémité distale du tube d'inspection, afin de modifier l'orientation de l'extrémité distale du tube d'inspection et donc de la fenêtre d'observation de la sonde, le dispositif électromécanique comprenant deux moteurs actionnant le béquillage distal par l'intermédiaire de deux paires respectives de câbles, pour modifier l'orientation suivant deux plans respectifs, de l'extrémité distale du tube d'inspection,
- un processeur délivrant deux signaux de commande appliqués respectivement aux deux moteurs, et
- des moyens d'introduction de commandes comportant deux organes d'introduction de commandes, connectés au processeur pour introduire des commandes destinées respectivement aux deux moteurs, chacun des deux organes de commande présentant un premier état dans lequel le processeur commande le moteur respectif de manière à maintenir fixe l'orientation de l'extrémité distale du tube d'inspection, un second et un troisième états dans lesquels le processeur commande le moteur respectif de manière à faire varier, respectivement dans un sens et dans un sens opposé, l'orientation de l'extrémité distale du tube d'inspection.

Avantageusement, chacun des moteurs est de type servomoteur actionnant une poulie couplée à une paire de câbles respective, et dont la position angulaire est commandée par le signal de commande respectif généré par le processeur et appliqué au moteur, chaque signal de commande présentant la forme d'un train d'impulsions, la largeur des impulsions correspondant à une position angulaire déterminée de la poulie, le processeur comprenant des moyens pour maintenir constante la largeur des impulsions de chaque signal de commande lorsque l'organe de commande respectif se trouve dans le premier état, et augmentant et diminuant la largeur des impulsions à une vitesse prédéfinie lorsque l'organe de commande respectif se trouve respectivement dans les second et troisième états.

De préférence, le vidéoendoscope comprend un organe de commande supplémentaire intégré dans le panneau de commande et connecté au processeur pour commander la largeur des impulsions des signaux de commande appliqués aux moteurs, de manière à ce qu'elle soit égale à une valeur médiane correspondant à une déformation nulle du béquillage distal.

Avantageusement, l'organe de commande supplémentaire est intégré aux moyens d'introduction de commandes du béquillage distal.

Selon un mode de réalisation de l'invention, les deux organes de commande comprennent chacun une paire de contacts qui sont ouverts dans le premier état, l'un ou l'autre des contacts étant fermé dans les second et troisième états.

Selon un mode de réalisation de l'invention, chacun des organes de commande comprend deux boutons poussoirs intégrés dans le panneau de commande, pour actionner respectivement les deux contacts qui sont à l'état ouverts au repos, et passent à l'état fermé pendant que le bouton poussoir respectif est maintenu enfoncé.

Selon un mode de réalisation de l'invention, les moyens d'introduction de commandes du béquillage comprennent un manche à balai capable d'actionner simultanément les deux organes d'introduction de commande.

De préférence, le vidéoendoscope comprend un organe de commande supplémentaire intégré au panneau de commande pour modifier la vitesse de variation de l'orientation de l'extrémité distale du tube d'inspection en sélectionnant une vitesse lente ou une vitesse rapide.

Alternativement, le processeur est programmé pour sélectionner une vitesse rapide de variation de l'orientation de l'extrémité distale du tube d'inspection si au moins un des deux organes de commande est maintenu dans le second ou troisième état pendant une durée supérieure à un seuil prédéfini, et pour sélectionner une vitesse lente de variation de l'orientation de l'extrémité distale du tube d'inspection si les deux organes de commande sont dans le premier état.

Avantageusement, le processeur est programmé pour déterminer l'orientation de l'extrémité distale du tube d'inspection en fonction de forme des signaux de commande appliqués respectivement aux deux moteurs, et pour afficher à l'écran d'affichage des symboles représentant l'orientation déterminée.

Subsidiairement, la présente invention a également pour but de proposer un coffret de rangement d'un tel vidéoendoscope, ne présentant pas les inconvénients de l'art antérieur précédemment décrit.

Cet objectif est atteint par le fait que le vidéoendoscope comprend un coffret de rangement et de transport renfermant une alimentation électrique et un générateur d'éclairage, le coffret de rangement comprenant un tambour autour duquel la sonde peut être enroulée, le tambour étant monté de manière à pouvoir tourner librement autour de son axe et comprenant une cavité tubulaire destinée à recevoir l'extrémité distale de la sonde, et débouchant tangentiellement à la surface cylindrique du tambour.

Avantageusement, le tambour comprend un moyeu central maintenu entre deux flasques coaxiales, la cavité étant réalisée à partir d'une des faces latérales du moyeu, et refermée latéralement par l'un des deux flasques.

De préférence, le moyeu central est réalisé dans une matière présentant un coefficient de frottement élevé.

Egalement de préférence, le moyeu central est réalisé dans une mousse alvéolaire dure.

Selon un mode de réalisation de l'invention, le coffret de rangement comprend un logement dans lequel est fixé le tambour, le logement présentant une ouverture donnant accès à une partie de la surface cylindrique du tambour.

Selon un mode de réalisation de l'invention, le coffret comprend un couvercle dont la face interne est recouverte de mousse, le tambour étant monté dans le coffret de manière à être bloqué en rotation par la mousse du couvercle lorsque ce dernier est refermé sur le coffret.

De préférence, le diamètre intérieur de la cavité cylindrique est légèrement supérieur au plus grand diamètre de l'extrémité distale de la sonde susceptible d'être enroulée autour du tambour.

Un mode de réalisation préféré de l'invention sera décrit ci-après, à titre d'exemple non limitatif, avec référence aux dessins annexés dans lesquels :
La figure 1 représente schématiquement en perspective un vidéoendoscope équipé d'une poignée de commande;
La figure 2 représente schématiquement en vue partielle en perspective la variante selon l'invention de la poignée de commande montrée sur la figure 1 ;
La figure 3 représente schématiquement sous la forme d'un schéma-bloc l'architecture interne du vidéoendoscope représenté sur la figure 1 ;
La figure 4 représente schématiquement sous la forme d'un schéma-bloc l'architecture interne d'une variante du vidéoendoscope représenté sur la figure 3 ;
La figure 5 montre plus en détail sous la forme d'un schéma-bloc une variante de l'architecture interne de la sonde vidéoendoscopique représentée sur la figure 1 ;
La figure 6 illustre schématiquement l'architecture d'un système vidéo simplifié associant la sonde vidéoendoscopique représentée sur la figure 1, à un générateur de lumière et à une source d'alimentation électrique ;
La figure 7 illustre schématiquement l'architecture d'un système vidéo complexe associant la sonde vidéoendoscopique représentée sur la figure 1, à un générateur de lumière et à un coffret externe d'alimentation intégrant un dispositif spécifique de traitement et de stockage d'images vidéo ;
La figure 8 représente un dispositif électromécanique d'actionnement d'un béquillage distal susceptible d'équiper la sonde vidéoendoscopique représentée sur la figure 1 ;
La figure 9 montre plus en détail une partie du dispositif électromécanique représenté sur la figure 8 ;
La figure 10 représente un circuit électronique de commande selon l'invention, du dispositif électromécanique illustré par les figures 8 et 9 ;
La figure 11 illustre un mode de représentation vidéo de l'orientation du béquillage distal.
La figure 12 représente schématiquement en perspective un coffret de rangement et de transport d'une sonde vidéoendoscopique, selon l'invention, la sonde étant montrée en dehors du coffret ;
La figure 13 représente schématiquement en perspective le coffret de rangement et de transport montré sur la figure 12 avec la sonde rangée dans le coffret;
La figure 14 montre en détail, en perspective partiellement éclatée, un tambour d'enroulement d'un tube d'inspection d'endoscope selon l'invention ;
Les figures 15 et 16 montrent respectivement en vues axiales et transversales le moyeu du tambour représenté sur la figure 14.

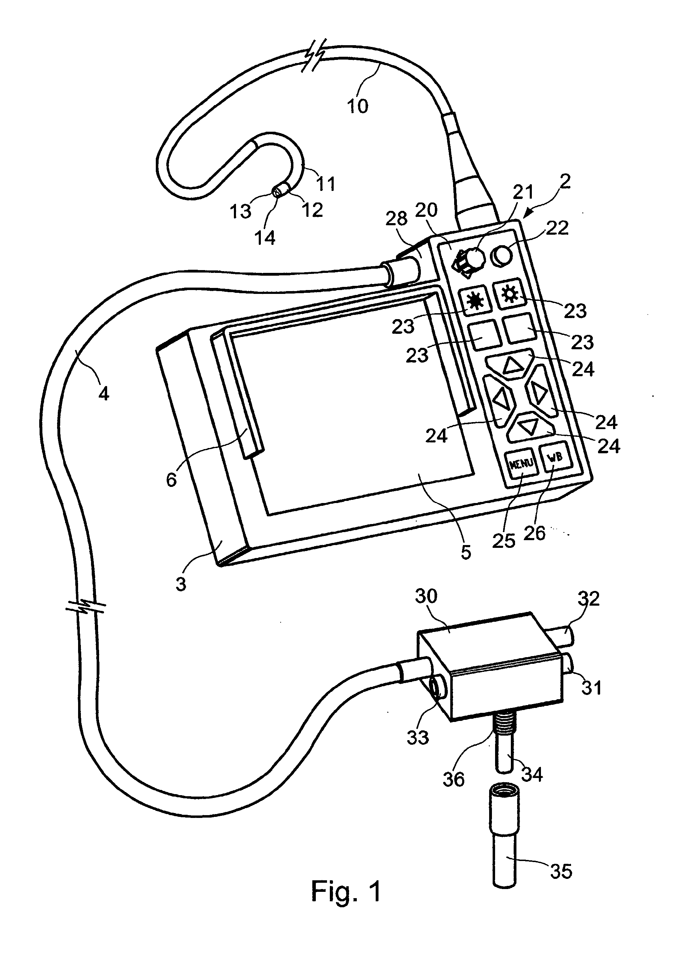

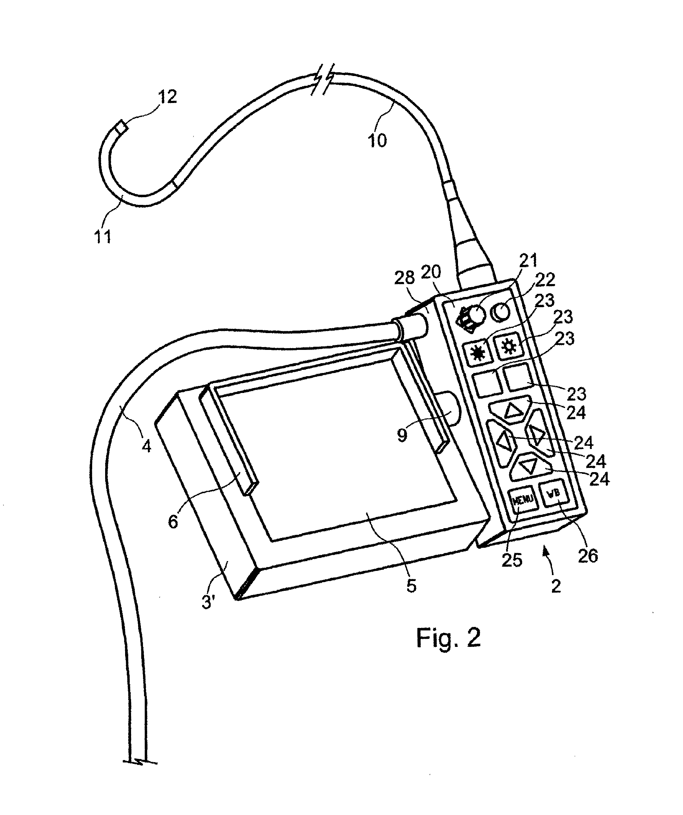

Les figures 1 et 2 représentent un vidéoendoscope comprenant une poignée de commande comportant un boîtier de commande 2 de forme sensiblement parallélépipédique allongée présentant une extrémité distale où vient se raccorder l'extrémité proximale d'une sonde d'inspection du vidéoendoscope.

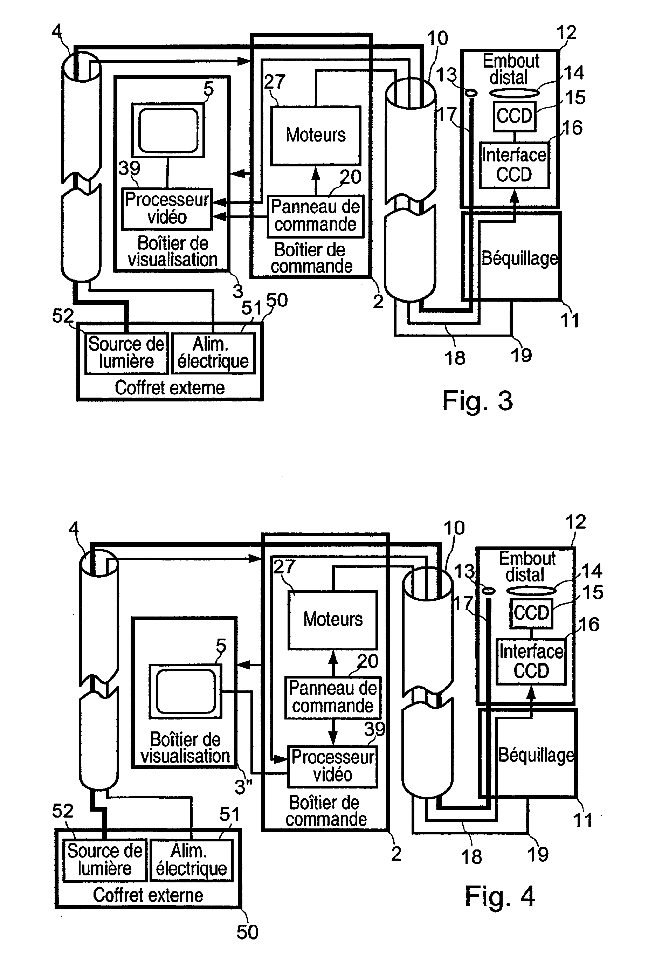

Le boîtier de commande 2 est également couplé à un câble ombilical 4 dont l'extrémité proximale est fixement solidaire d'un dispositif de connexion 30 permettant de raccorder la sonde d'endoscope à une source de lumière 52 et à une source d'énergie électrique 51 (voir figure 3).

La sonde d'inspection comprend un tube d'inspection 10 et un embout distal 12 relié à l'extrémité distale du tube d'inspection. L'embout distal 12 renferme classiquement un dispositif d'imagerie comprenant un objectif distal 14 formant une image réelle de la cible observée sur la couche photosensible d'un capteur CCD 15 (voir figure3) de préférence couleur, auquel l'objectif est associé. Le capteur CCD est couplé à un microcircuit d'interface 16 destiné à corriger les signaux électriques reçus ou générés par le capteur CCD. Le circuit d'interface 16 est relié à la poignée de commande 2 par un câble multiconducteurs 18 passant dans le tube d'inspection 10 et dans lequel transitent les tensions d'alimentation du dispositif d'imagerie, les signaux de synchronisation du capteur CCD et le signal électrique délivré par ledit capteur.

La poignée de commande comprend également un boîtier de visualisation 3 solidaire du boîtier de commande 2, renfermant un processeur vidéo 39 relié au dispositif d'imagerie par le câble de liaison multiconducteurs 18 passant dans le tube d'inspection 10, et un écran d'affichage 5, par exemple du type moniteur vidéo éventuellement couleur, commandé par le processeur vidéo et logé dans le boîtier de visualisation. L'écran d'affichage 5 est de préférence plat et de faible poids, par exemple de type LCD (Liquid Crystal Display) et le boîtier de visualisation 3 présente des dimensions correspondant sensiblement à celles de l'écran d'affichage.

On peut prévoir de surélever le bord supérieur et une partie supérieure des bords latéraux de l'écran d'affichage 5 de manière à former un pare soleil 6.

Le boîtier de commande 2 comprend une face supérieure sur laquelle est disposé un panneau de commande 20 rassemblant des touches 23 à 26 de commande de l'endoscope et du moniteur vidéo 5.

Plus précisément, le panneau de commande 20 rassemble les touches suivantes :
- un ensemble de touches 23 permettant de régler directement les principaux paramètres du processeur vidéo 39 et du moniteur vidéo 5 (notamment la luminosité et le contraste),
- une touche 25 d'accès à un menu de sélection de fonctions de réglage de l'ensemble des paramètres de fonctionnement du processeur vidéo,
- quatre touches directionnelles 24 de navigation permettant de sélectionner des options dans les différents menus accédés à l'aide de la touche 25, et
- une touche 26 de commande de la balance des blancs qui est appliquée au processeur vidéo.

La face distale de l'embout 12 dispose également d'une fenêtre d'éclairage 13 en regard de l'extrémité distale d'un faisceau continu de fibres d'éclairage 17 logé dans la sonde d'inspection 10, dans la poignée de commande 2, puis dans le câble ombilical 4, de manière à relier sans rupture de continuité, la fenêtre d'éclairage 13 à l'extrémité proximale du câble ombilical 4 solidaire d'un embout de connexion d'éclairage 34 logé dans le boîtier de connexion 30 de la sonde vidéoendoscopique. La fenêtre d'éclairage 13 est disposée sur l'embout distal 12 de manière à illuminer la cible située dans le champ de la fenêtre optique 14, lorsque l'embout d'éclairage 34 du dispositif de connexion 30 est connectée sur un générateur de lumière 52 (figure 3).

Par ailleurs, la sonde d'inspection peut comprendre un béquillage distal articulé 11 dont les déformations sont commandées par des câbles 19 logés dans le tube d'inspection 10 et actionnés par une carte de commande 27 intégrant des moteurs, logée dans le boîtier de commande 2 (figure 3). Dans ce cas, le boîtier de commande 2 intègre un dispositif de commande 21 à quatre degrés de liberté, par exemple du type manche à balai, reliée à la carte de commande pour commander les moteurs.

Selon une première variante, le boîtier de visualisation 3 est solidarisé sur une face latérale 28 du boîtier de commande 2.

De cette manière, l'utilisateur peut tenir l'ensemble du boîtier de commande 2 et du boîtier de visualisation 3 à l'aide de ses deux mains, une main maintenant le boîtier de commande et l'autre le boîtier de visualisation. Du fait de la forme allongée du boîtier de commande 2, l'utilisateur peut à la fois maintenir l'ensemble du boîtier de commande et du boîtier de visualisation 3, et accéder à l'ensemble des touches du panneau de commande 20 à l'aide de son pouce.

En outre, les dimensions de l'écran d'affichage 5 peuvent être notablement plus importantes qu'avec les poignées de commande de l'art antérieur.
Grâce à l'invention, il n'est ainsi plus nécessaire de recourir systématiquement à un moniteur vidéo externe, ce qui renforce les avantages du vidéoendoscope en ce qui concerne son autonomie de fonctionnement, sa mobilité et sa simplicité et facilité de mise en oeuvre et d'utilisation.

De préférence, le câble ombilical 4 est raccordé au boîtier de commande 2 par une face latérale de celui-ci. De cette manière, l'utilisateur est moins gêné par ce câble. Bien entendu, la zone de raccordement du câble ombilical 4 sur le boîtier de commande 2 peut être sur la face latérale 28 où est solidarisé le boîtier de visualisation 3 ou sur l'autre face latérale du boîtier de commande 2.

De préférence, la face latérale 28 est située à la main gauche de l'utilisateur qui peut alors actionner le panneau de commande 20 du boîtier de commande 2 à l'aide de sa main droite.

Selon la variante de l'invention représentée sur la figure 2, le boîtier de visualisation 3' est solidarisé avec le boîtier de commande 2 par une articulation 9 permettant d'orienter l'écran 5 par rapport au boîtier de commande 2 autour d'un axe perpendiculaire à la face latérale 28 de celui-ci. Cette disposition permet d'orienter l'écran 5 en fonction de sa position par rapport aux yeux de l'utilisateur, et pour en supprimer les reflets.
Dans ce cas, tous les fils de raccordement électrique du boîtier de visualisation passent avantageusement par l'articulation 9.

Dans les deux variantes décrites ci-avant, on peut prévoir que le boîtier de visualisation 3, 3' soit démontable et déconnectable du boîtier de commande 2. Dans ce cas, le boîtier de visualisation est enfichable latéralement sur le boîtier de commande 2.

Dans la seconde variante de l'invention, l'articulation 9 peut être constituée d'un connecteur assurant à la fois la connexion électrique et la fixation mécanique du boîtier de visualisation 3' latéralement sur le boîtier de commande 2, tout en autorisant le pivotement de l'un par rapport à l'autre et la séparation des deux boîtiers.

Dans les deux variantes, le boîtier de commande 2 peut comprendre sur ses deux faces latérales des moyens d'accouplement mécanique et électrique pour la fixation et la connexion électrique du boîtier de visualisation 3, 3' indifféremment sur l'une ou l'autre des faces latérales du boîtier de commande, selon que l'utilisateur est droitier ou gaucher.

Conformément à une autre variante de l'invention représentée sur la figure 4, le processeur vidéo est logé dans le boîtier de commande 2. De cette manière, le boîtier de commande 2 selon l'invention peut être utilisé soit avec un boîtier de visualisation 3" avec lequel il est couplé électriquement et mécaniquement de manière amovible, soit avec un moniteur vidéo externe par l'intermédiaire d'un câble de connexion.

Il est à noter que du fait que le processeur vidéo est disposé dans la poignée de commande, le câble électrique multiconducteurs reliant le processeur vidéo au circuit d'interface CCD 16 est de longueur fixe. Il en résulte que le processeur vidéo peut être réglé en usine en fonction de la longueur du câble.

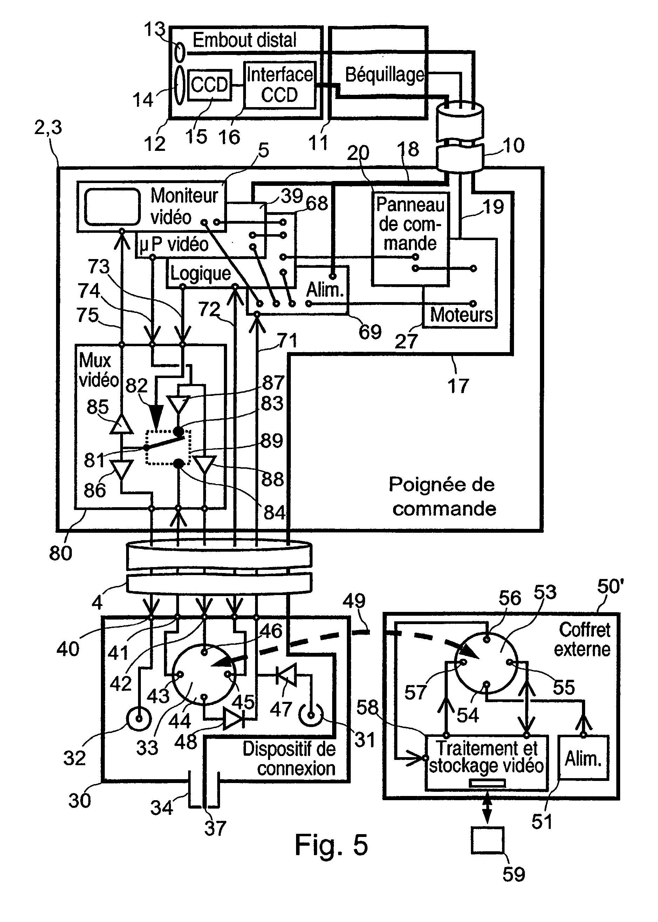

Tel que représenté sur la figure 5, la poignée de commande 2 loge également un multiplexeur vidéo 80 permettant de visualiser sur l'écran vidéo 5 soit l'image délivrée par le processeur vidéo, soit des images vidéo délivrées par un dispositif 58 de traitement et/ou de stockage d'images, susceptible d'être raccordé au dispositif de connexion 30, par l'intermédiaire d'un câble de raccordement 49.

Le câble ombilical 4 loge d'une part le faisceau de fibres d'éclairage 17 de la sonde vidéoendoscopique, et d'autre part, un câble électrique multiconducteurs dans lequel transitent une tension électrique d'alimentation générale de la poignée de commande 2, un signal vidéo délivré par le multiplexeur vidéo 80, des signaux logiques de commande échangés entre la poignée de commande 2 et un dispositif 58 de traitement et/ou de stockage d'images vidéo susceptible d'être connecté au dispositif de connexion 30, et un signal vidéo délivré par le dispositif de traitement et/ou de stockage.

Le dispositif de connexion 30 est équipé d'un embout de connexion cylindrique 34 raccordé à l'extrémité proximale du faisceau de fibres d'éclairage de la sonde vidéoendoscopique. L'embout de connexion 34 comprend un filetage 36 permettant de visser un adaptateur mécanique 35 correspondant au standard mécanique du connecteur d'éclairage d'un générateur de lumière 52 choisi par l'utilisateur. Le dispositif de connexion 30 comprend également une embase de connecteur 31 destinée à être connectée à une source électrique d'alimentation à tension continue, une embase de connecteur de sortie vidéo 32 recevant le signal vidéo en sortie du multiplexeur vidéo logé dans la poignée de commande 2, et destinée à être raccordée à un moniteur vidéo auxiliaire, et une embase de connecteur multibroches 33 destinée à être connectée à un dispositif externe 58 de traitement et/ou de stockage d'images vidéo.

Les touches tactiles 23, 24, 25 du panneau de commande 20 permettent à l'utilisateur de gérer aussi bien les fonctions vidéo du processeur vidéo logé dans la poignée de commande 2, que les fonctions du dispositif 58 de traitement et/ou de stockage d'images susceptible d'être raccordé à l'embase de connecteur multibroches 33 du dispositif de connexion 30. La touche tactile 26 permet de régler automatiquement la balance des blancs du processeur vidéo 39, en fonction des caractéristiques spectrales de l'éclairage délivré par la fenêtre d'illumination 13 de l'embout distal 12 du tube d'inspection 10, et donc in fine des caractéristiques spectrales de la lampe du générateur de lumière sur lequel est connecté le connecteur d'éclairage 34 du dispositif de connexion 30.

Plus précisément sur la figure 5, la poignée de commande 2 loge les éléments suivants :
- l'ensemble électromécanique 27 regroupant deux servomoteurs actionnant des câbles 19 de commande du béquillage 11 situé à l'extrémité distale du tube d'inspection 10, ainsi que des dispositifs électroniques de gestion desdits servomoteurs ;
- le processeur vidéo 39 relié au capteur CCD 15 distal de la sonde vidéoendoscopique par un câble multiconducteurs 18 logé dans le tube d'inspection 10 et dans lequel transitent les signaux de synchronisation du capteur CCD, générés par le processeur vidéo, le signal électrique généré par le capteur CCD et les alimentations électriques du capteur CCD, le processeur vidéo délivrant un signal vidéo standard sur une liaison 74 qui est connectée à une entrée du multiplexeur vidéo 80 ;
- l'écran vidéo 5 relié par une liaison 75 à une sortie du multiplexeur 80 et permettant de visualiser le signal vidéo délivré par le multiplexeur vidéo ;
- une carte logique 68 générant sur une liaison 73 un signal de commande du multiplexeur vidéo 80, ainsi que les signaux de commande du processeur vidéo 39 et de l'écran vidéo 5, et dans une liaison 72 les signaux de commande du dispositif 58 de traitement et de stockage externe, en fonction des commandes introduites à l'aide du panneau de commande 20 ;
- le panneau de touches de commandes 20 générant les ordres destinés d'une part aux dispositifs de gestion des servomoteurs de commande de béquillage et d'autre part à la carte logique 68 ; et
- un circuit d'alimentation 69 recevant par une liaison 71 une tension d'alimentation externe et générant les alimentations nécessaires aux différents éléments 5, 27, 39, 68 de la poignée de commande 2, ainsi que les tensions d'alimentation nécessaires au dispositif d'imagerie intégré dans l'embout distal 12 du tube d'inspection 10.

Le multiplexeur vidéo 80 comprend un commutateur 89 à deux entrées 83 et 84, une sortie 81 et une entrée de commande 82. Une première entrée 83 du commutateur 89 reçoit, à travers un amplificateur 87 le signal vidéo 74 délivré par le processeur vidéo 39. La seconde entrée 84 du commutateur 89 reçoit le signal vidéo délivré par le dispositif externe de traitement et de stockage d'images. La sortie 81 du commutateur 89 distribue le signal vidéo sélectionné par le commutateur 89 vers l'écran vidéo 5 à travers un amplificateur 85, et à travers un amplificateur 86 et une liaison coaxiale 40, vers l'embase de connecteur de sortie vidéo 32 du boîtier de connexion 30. L'entrée de commande 82 du commutateur reçoit par la liaison 73 le signal logique délivré par la carte logique 68.

Le dispositif de connexion 30 loge l'extrémité proximale 37 du faisceau de fibres d'éclairage 17 de la sonde vidéoendoscopique.

L'embase de connecteur 31 de raccordement à une alimentation auxiliaire est relié par l'intermédiaire d'une diode 47 et du câble 71 à la carte d'alimentation 69 logée dans la poignée de commande.

L'embase de connecteur 33 comprend quatre broches 43, 44, 45, 46, et peut être raccordée, comme représenté sur la figure 5, par un câble 49, à une embase de connecteur 53 à quatre broches 54, 55, 56, 57 prévue sur un coffret externe 50'. La broche d'alimentation 44 est reliée, à travers une diode 48 et le câble 71 à la carte d'alimentation 69 logée dans la poignée de commande. La broche 45 est reliée via la liaison 72 à la carte logique 68 intégrée dans la poignée de commande 2, pour permettre la circulation des signaux de commande émis par la carte logique 68 intégrée dans la poignée de commande 2, vers le dispositif externe 58 de traitement d'images vidéo. La broche 46 reçoit, via une liaison 42 et un amplificateur 88 du multiplexeur vidéo 80, le signal vidéo 74 délivré par le processeur vidéo 39 logé dans la poignée de commande 2. La broche 43 délivre le signal vidéo généré par le dispositif de traitement d'image 58 sur la liaison 41 reliée à l'entrée 84 du commutateur vidéo 89.

Avantageusement, l'embase de connecteur 33 comprend une broche supplémentaire (non représentée) qui est reliée à la carte logique 68, pour déclencher l'activation du multiplexeur 80 dès qu'un système de traitement d'image est connecté à l'embase de connecteur 33.

Bien entendu, le multiplexeur vidéo 80 peut être alternativement intégré dans le boîtier 3 de visualisation ou dans le boîtier de connexion 30. Dans ce dernier cas, le processeur vidéo 39 peut également être intégré dans le boîtier de connexion.

La sonde vidéoendoscopique qui vient d'être décrite, munie du multiplexeur 80 et du dispositif de connexion 30, peut être connectée indifféremment à n'importe quel générateur de lumière en utilisant un adaptateur 35 correspondant à l'embase de connexion du générateur. Elle peut également être raccordée à un moniteur vidéo auxiliaire et à un système 58 de traitement et/ou de stockage d'images, tout en offrant la possibilité de visualiser les images enregistrées par un tel système à l'écran de la poignée de commande, et de piloter ce système de cette dernière. Ainsi, les quatre touches de navigation 24 (flèches dans les quatre directions) de la poignée de commande peuvent être configurées pour commander en fonctionnement normal l'enregistrement de l'image visualisée à l'écran 5 (flèche vers le haut), l'effacement de la dernière image enregistrée (flèche vers le bas), l'affichage à l'écran 5 de l'image enregistrée précédente (flèche vers la gauche) et suivante (flèche vers la droite).

Ces dispositions confèrent à la sonde endoscopique selon l'invention une grande souplesse et ergonomie d'utilisation.

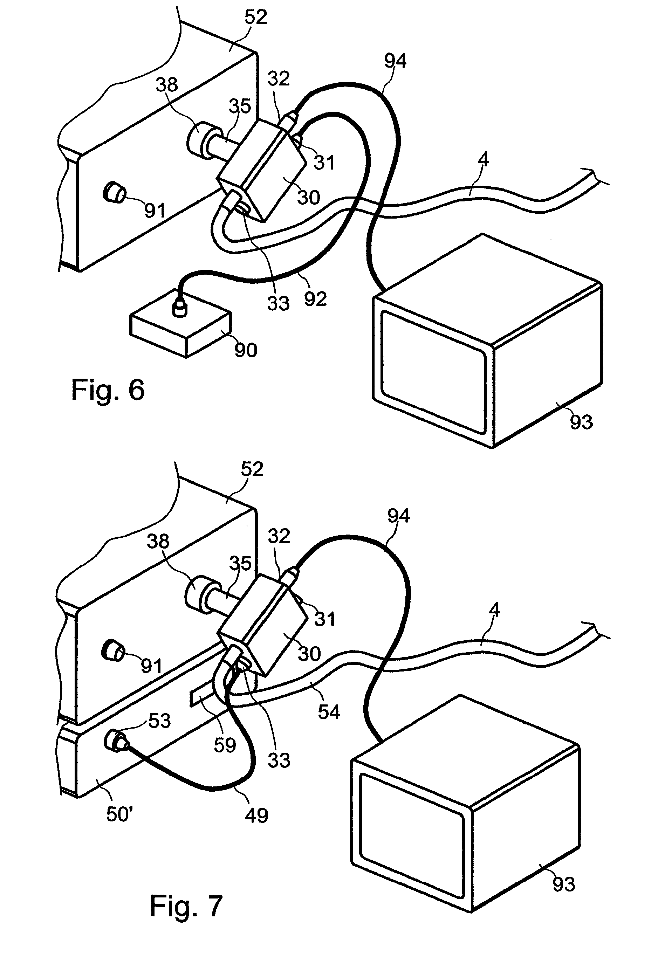

Ainsi, comme cela est représenté sur la figure 6, la sonde vidéoendoscopique selon l'invention peut être intégrée dans un système vidéo simple comprenant un générateur de lumière 52 et une source d'alimentation électrique 90 à tension continue, banalisée.

A cet effet, l'adaptateur 35 est vissé sur l'embout de connexion d'éclairage 34 du dispositif de connexion 30 et est enfiché dans un connecteur d'éclairage 38 du générateur de lumière 52 dont le niveau d'éclairement peut être dosé par rotation d'un bouton de commande de diaphragme 91. Par ailleurs, le connecteur d'alimentation électrique 31 du dispositif de connexion 30 est relié par l'intermédiaire d'un cordon 92 à l'alimentation électrique externe 90.

L'embase de connecteur de sortie vidéo 32 du dispositif de connexion 30 peut être reliée par l'intermédiaire d'un cordon coaxial 94, à un moniteur vidéo auxiliaire 93 permettant de visualiser le signal vidéo généré par le processeur vidéo intégré dans la poignée de commande de la sonde vidéoendoscopique, l'image diffusée par le moniteur auxiliaire 93 étant alors identique à celle affichée à l'écran vidéo 5 de la poignée de commande.

Tel que cela est représenté sur la figure 7, la sonde vidéoendoscopique selon l'invention peut être intégrée dans un système vidéo plus complexe comprenant un générateur de lumière 52 et un coffret externe 50' intégrant, comme cela est montré plus en détail sur la figure 5, une alimentation électrique continue 51 et un dispositif 58 de traitement et/ou de stockage d'images vidéo sur une carte mémoire 59, de préférence amovible.

A cet effet, l'adaptateur 35 vissé sur l'embout de connexion d'éclairage 34 du dispositif de connexion 30 est enfiché dans le connecteur d'éclairage 38 du générateur de lumière 52 dont le niveau d'éclairement peut être dosé par rotation du bouton de commande de diaphragme 91.

L'embase de connecteur multibroches 33 du dispositif de connexion 30 est quant à elle reliée par l'intermédiaire du cordon multiconducteurs 49 à l'embase de connecteur multibroches 53 du coffret externe 50'.

Dans le coffret externe 50' représenté plus en détail sur la figure 5, la broche 54 du connecteur multibroches 53, destinée à être raccordée à la broche 44 de l'embase de connecteur 33, est connectée au circuit d'alimentation électrique 51. La broche 56 destinée à être raccordée à la broche 46, est connectée à une entrée vidéo du dispositif 58 de traitement et de stockage d'images vidéo. La broche 57 destinée à être raccordée à la broche 43, est connectée à une sortie vidéo du dispositif 58. La broche 55 destinée à être raccordée à la broche 45, est connectée à une entrée/sortie de commande logique du dispositif 58 de dialogue.

Dans le cordon 49 transitent donc les signaux suivants :
- le signal vidéo généré par le processeur vidéo intégré dans la poignée de commande 2,
- le signal vidéo généré par le dispositif 58 de traitement et/ou de stockage intégré dans le coffret externe 50',
- la tension d'alimentation générée par le coffret externe 50', et
- les signaux de commande du coffret 50' générés par la poignée de commande à la suite de l'activation par l'utilisateur des touches 24 du panneau de commande 20.

Comme pour le système représenté sur la figure 6, un moniteur vidéo auxiliaire 93 peut être raccordé par l'intermédiaire d'un cordon coaxial 94 à l'embase de connecteur de sortie vidéo 32 du dispositif de connexion 30, pour visualiser sur ce moniteur auxiliaire les images vidéo délivrées par le multiplexeur vidéo intégré dans la poignée de commande, ces images étant identiques à celles affichées à l'écran vidéo 5 de la poignée de commande.

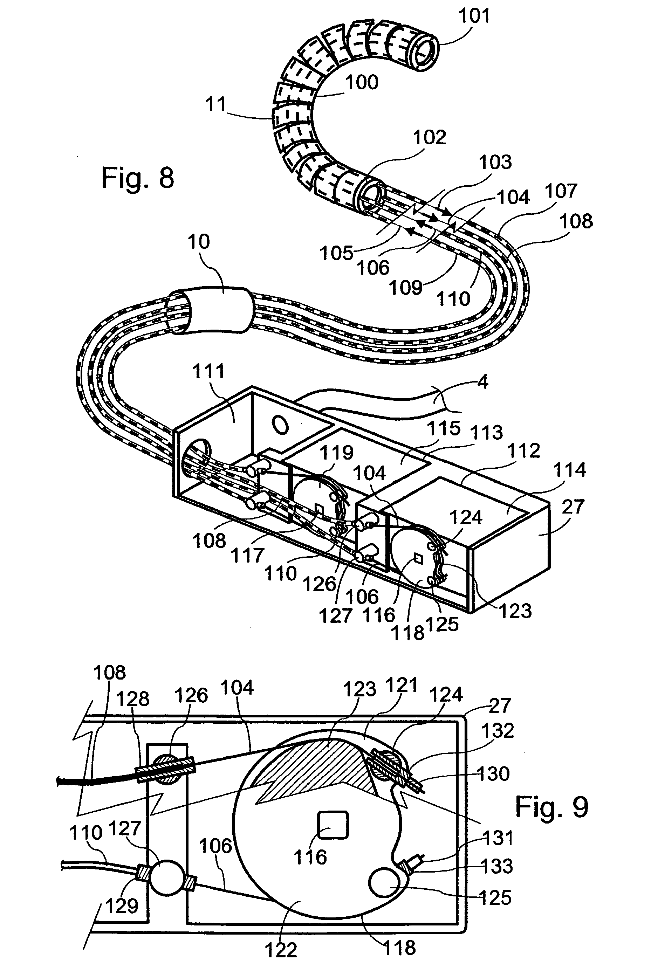

Sur la figure 8, l'orientation du béquillage distal 11 dans deux plans perpendiculaires est commandée par deux paires de câbles de manoeuvre 103, 105 et 104, 106 logés dans le tube d'inspection 10 et actionnés par un dispositif électromécanique 27 logé dans la poignée de commande 2.

Ce dispositif comprend deux servomoteurs 114, 115, et un dispositif de commande des servomoteurs. Les deux servomoteurs 114, 115 entraînent en rotation, par l'intermédiaire d'un axe respectif 116, 117 des poulies respectives 118, 119, actionnant deux paires de câbles respectives 103, 105 et 104, 106, les extrémités distales des deux paires de câbles étant fixées au béquillage 11 pour commander l'orientation de celui-ci dans deux plans respectifs, perpendiculaires l'un à l'autre.

Les servomoteurs 114, 115, et le dispositif de commande des servomoteurs sont logés dans la poignée de commande 2 qui comprend à cet effet trois compartiments 111, 112, 113. Le compartiment 111, solidaire de l'extrémité proximale du tube d'inspection 10, loge le circuit de commande des servomoteurs 114, 115, tandis que les deux autres compartiments 112, 113 logent respectivement les servomoteurs 114, 115.

Les compartiments 112 et 113 sont avantageusement décalés transversalement de façon à ce que les poulies 118 et 119 soient convenablement positionnées dans deux plans parallèles.

Le béquillage distal 11 de la sonde d'inspection 10 est constitué d'une série d'anneaux 100 reliés les uns aux autres par des articulations autorisant une déformation dudit béquillage permettant d'orienter son extrémité distale dans deux plans perpendiculaires. Le dernier anneau constituant l'extrémité distale du béquillage 2 est fixement solidaire d'une bague 101 percée de quatre trous longitudinaux disposés à 90° les uns des autres et dans lesquels sont fixées les extrémités distales des quatre câbles d'actionnement 103, 104, 105, 106. Le dernier anneau constituant l'extrémité proximale du béquillage 11 est également fixement solidaire d'une bague 102 percée de quatre trous longitudinaux disposés à 90° les uns des autres et dans lesquels glissent librement les quatre câbles 103 à 106. La face proximale de ladite bague sert par ailleurs de butée fixe à quatre gaines souples 107, 108, 109, 110 dans lesquelles coulissent respectivement les quatre câbles 103 à 106.

Tel que représenté sur la figure 8 et montré plus en détail sur la figure 9, la poulie 118 entraînée par l'axe 116 du servomoteur 114 et actionnant la paire de câbles 104, 106, comprend un noyau central 123 solidaire de deux flasques latérales débordantes 121, 122 dans les parties proximales desquelles sont ménagés des orifices circulaires transversaux dans lesquels sont librement logées deux pièces cylindriques 124, 125 dont les parties centrales sont fixement solidaires des extrémités distales des câbles 104, 106.
La poulie 118 est par ailleurs associée à deux pièces cylindriques de guidage 126, 127 montés librement rotatifs dans deux orifices cylindriques ménagés à cet effet dans une cloison distale du logement 112 renfermant le servomoteur 114 dont l'axe 116 entraîne ladite poulie. Ces pièces de guidage disposant d'orifices transversaux dans lesquels circulent librement les câbles 104 et 106, servent de butées réglables aux gaines souples 108, 110 logeant respectivement les câbles 104, 106.

Des dispositifs analogues de guidage et de butée sont associés à la poulie 119 solidaire de l'axe 117 du moteur 115 logé dans le compartiment 113 et actionnant la paire de câbles 103, 105.

Plus précisément, la pièce cylindrique 124, librement logée dans deux orifices circulaires ménagés à cet effet dans la partie proximale des flasques 121, 122 associées au noyau 123, dispose d'un orifice fileté transversal traversant, dans lequel est vissée une pièce tubulaire 132, cylindrique, comportant un canal distal dans lequel est librement logé le câble 104, et un canal proximal coaxial plus étroit servant également de logement au câble 104 et dont la face proximale sert de butée à un embout 130 fixement soudé autour de l'extrémité distale du câble 104.

La pièce cylindrique 126, librement logée dans un orifice cylindrique ménagé à cet effet dans la cloison distale du compartiment 112 dans lequel est logé le moteur actionnant l'axe 116, dispose d'un orifice fileté transversal traversant dans lequel est vissée une pièce cylindrique 128. Cette pièce 128 présente une forme tubulaire avec un canal proximal dans lequel est librement logé le câble 104 et un canal distal coaxial, plus large, servant de logement à la gaine 108 et dont l'extrémité proximale sert de butée à ladite gaine.

Des dispositifs analogues (pièce cylindrique 134, pièce tubulaire filetée 133, embout soudé 131) permettent de régler la fixation du câble 106 et de régler (pièce cylindrique 127, pièce tubulaire filetée 129) la butée de la gaine 110.

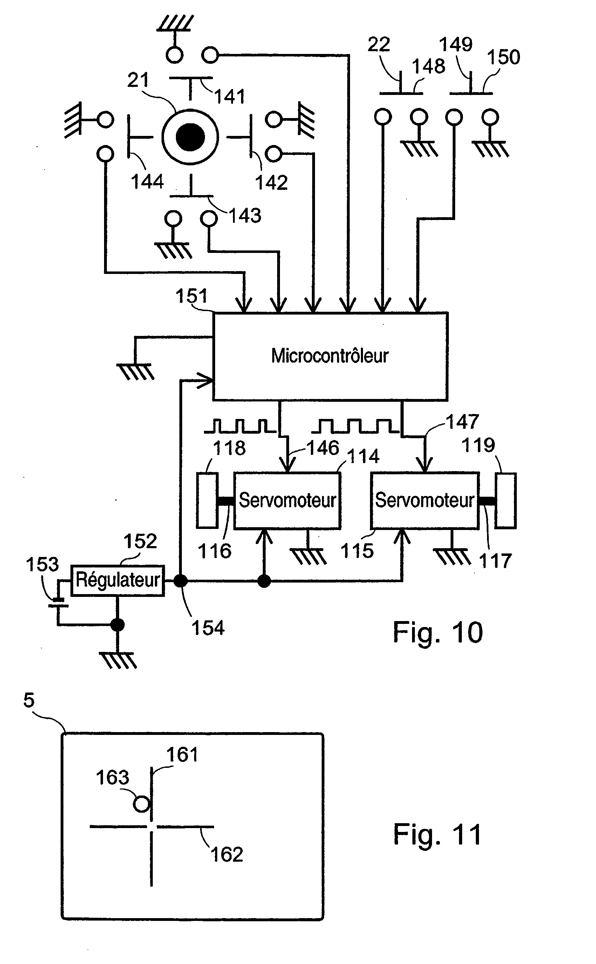

Les deux servomoteurs 114, 115 sont pilotés par un dispositif de commande selon l'invention, tel que représenté sur la figure 10. Sur cette figure, le dispositif de commande comprend un processeur 151, par exemple de type microcontrôleur, programmé pour délivrer deux signaux 146, 147 respectifs de commande des servomoteurs 114, 115.

Le microcontrôleur 151 est connecté aux éléments électromécaniques de commande suivants :
- deux contacts 142, 144 commandant respectivement l'augmentation et la diminution de la largeur des impulsions 146 appliquées au moteur 114, de manière à agir sur l'orientation du béquillage 11 dans un premier plan, et
- deux contacts 141, 143 commandant respectivement l'augmentation et la diminution de la largeur des impulsions 147 appliquées au moteur 115, de manière à agir sur l'orientation du béquillage 11 dans un second plan perpendiculaire au premier,

Les contacts 141 à 144 peuvent être actionnés par des boutons poussoirs respectifs à action fugitive (par exemple 24 sur la figure 1), c'est-à-dire que les contacts sont ouverts au repos et passent à l'état fermé pendant que le bouton poussoir respectif est maintenu enfoncé.
Alternativement, on peut prévoir avantageusement un manche à balai 21 (communément appelé joystick) qui actionne par basculement ces contacts 141 à 144. Pour actionner les paires de contacts 141, 143 et 142, 144, on peut alternativement prévoir deux boutons basculants à trois états respectifs, à savoir une position neutre dans laquelle les deux contacts de la paire respective sont à l'état ouvert, et deux états dans chacun desquels l'un ou respectivement l'autre des deux contacts de la paire est fermé.

Les moteurs 114, 115 sont des servomoteurs traditionnels alimentés en tension continue 154 (égale par exemple à 5V) délivrée par un régulateur 152 lui même alimenté par une tension continue 153 (égale par exemple à 12V). Par ailleurs, les servomoteurs sont commandés à l'aide de signaux de commande présentant la forme de trains d'impulsions à basse fréquence constante, par exemple 50 Hz.

Le dispositif de commande décrit ci-avant fonctionne de la manière décrite ci-après.
L'axe mécanique du servomoteur 114 (ou 115), et donc la poulie 118 (respectivement 119) solidaire dudit axe, présente une course angulaire globale α par nature limitée (il s'agit d'une caractéristique propre aux servomoteurs), α étant par exemple égal à 160°. Une des positions angulaires extrêmes de la poulie 118 (respectivement 119), à un angle de 0°, correspondant à une déformation maximale dans une direction du béquillage distal, est obtenue quand le microcontrôleur 151 transmet au servomoteur 114 (respectivement 115) un train d'impulsions 146 (respectivement 147) de largeur minimum (d'une durée égale par exemple à 0,8 ms). L'autre position angulaire extrême de la poulie 118 (respectivement 119) égale à α, correspondant à une déformation maximale du béquillage distal dans la direction opposée à la précédente, est obtenue quand le microcontrôleur 151 transmet au servomoteur 114 (respectivement 115) un train d'impulsions 146 (respectivement 147) de largeur maximum (d'une durée égale par exemple à 2,2 ms). La position angulaire médiane de la poulie 118 (respectivement 119), égale à α/2, correspondant à une orientation neutre du béquillage distal, est obtenue lorsque le microcontrôleur 151 transmet au servomoteur 114 (respectivement 115) un train d'impulsions 146 (respectivement 147) de largeur moyenne (d'une durée égale à 1,5 ms pour 2,2 ms de durée maximum).

Les variations de la position angulaire de la poulie 118 (respectivement 119) à partir d'une position initiale sont commandées dans un sens par la fermeture du contact 142 (respectivement 141), et dans l'autre sens par la fermeture du contact 144 (respectivement 143).
Lorsque les deux contacts 142, 144 (respectivement 141, 143) sont ouverts, la poulie 148 (respectivement 149) est immobilisée dans une position angulaire correspondant à la largeur des impulsions 146 (respectivement 147) appliquées par le microcontrôleur 151 au moteur 114 (respectivement 115), lors des précédentes manipulations des contacts 142, 144 (respectivement 141, 143).

Si l'on ferme le contact 142 (ou 141), le microcontrôleur commande alors une augmentation progressive de la largeur des impulsions 146 (respectivement 147), jusqu'à la réouverture du contact 142 (respectivement 141) ou jusqu'à atteindre la valeur maximum de cette largeur.

Dans l'autre sens, si l'on ferme le contact 144 (ou 143), le microcontrôleur commande une diminution progressive de la largeur des impulsions 146 (respectivement 147), jusqu'à la réouverture du contact 144 (respectivement 143) ou jusqu'à atteindre la valeur minimum de cette largeur. Il en résulte que tant que la largeur des impulsions n'atteint pas sa valeur minimum ou maximum, l'amplitude de variation de cette largeur est proportionnelle à la durée de fermeture du contact correspondant.
La vitesse de variation de la largeur des impulsions 146 (ou 147), et donc la vitesse de rotation de la poulie 118 (respectivement 119), et donc la vitesse de variation de l'orientation du béquillage 11, provoquée par la fermeture du contact 142 ou 144 (respectivement 141 ou 143) est fixée par programmation du microcontrôleur 151.

La génération des trains d'impulsions de commande 146, 147 des servomoteurs 114, 115 par un microcontrôleur, permet de connaître à tout instant les largeurs des impulsions appliquées aux servomoteurs et donc la position angulaire des poulies entraînées par les servomoteurs. Il est donc facile de déterminer à tout instant l'orientation du béquillage. Cette orientation peut ainsi être affichée à l'écran 5, par exemple à l'aide d'un symbole 163 représentant l'extrémité distale du béquillage, positionné par rapport à deux axes 161, 162 pour indiquer une déviation horizontale et une déviation verticale du béquillage (figure 11).

Ces dispositions permettent en outre d'ajuster la vitesse de déplacement du béquillage distal 11. Il est en effet commode de disposer d'une vitesse rapide de déplacement du béquillage distal pour balayer rapidement la zone observée au moyen du vidéoendoscope, et d'une vitesse lente pour centrer précisément à l'écran le détail que l'on veut observer.

Pour modifier la vitesse de déplacement du béquillage distal et donc la vitesse de variation de la largeur des impulsions appliquées aux servomoteurs, on peut prévoir un bouton poussoir 149 à enclenchement, agissant sur un contact 150 connecté au microcontrôleur 151. Sur détection de la fermeture du contact 150, le microcontrôleur sélectionne une vitesse rapide de variation de la largeur des impulsions 146, 147, ce qui augmente la vitesse de variation de l'orientation du béquillage distal. Inversement, si l'on appuie à nouveau sur le bouton poussoir 149, le contact 150 revient en position ouverte. Le microprocesseur sélectionne alors la vitesse lente qui sera utilisée pour modifier l'orientation du béquillage à la suite de la détection de la fermeture d'un des contacts 141 à 144.

L'activation de la vitesse de variation rapide de la largeur des impulsions à partir de la vitesse lente peut également être déclenchée automatiquement par le microcontrôleur si l'un des contacts 141 à 144 est détecté fermé par le microcontrôleur 151 pendant une durée supérieure à un seuil prédéfini, par exemple de l'ordre de une à deux secondes. Dès que le microcontrôleur détecte que les contacts 141 à 144 sont tous à l'état ouvert, la vitesse lente est automatiquement sélectionnée pour exécuter une commande suivante de modification de l'orientation du béquillage.

Par ailleurs, on peut prévoir sur le panneau de commande 20 un bouton poussoir 22 de mise en position neutre du béquillage 11, agissant sur un contact 148 à action fugitive, connecté au microcontrôleur, pour fixer la largeur des impulsions 146, 147 à une même valeur moyenne, correspondant au milieu de la course des poulies 118, 119 (positionnement angulaire des poulies 118, 119 à un angle égal à α/2), annulant les déformations appliquées au béquillage distal 11. La commande d'annulation des déformations appliquées au béquillage peut avantageusement être intégrée au manche à balai 21 de manière à être déclenchée en appuyant sur le manche.

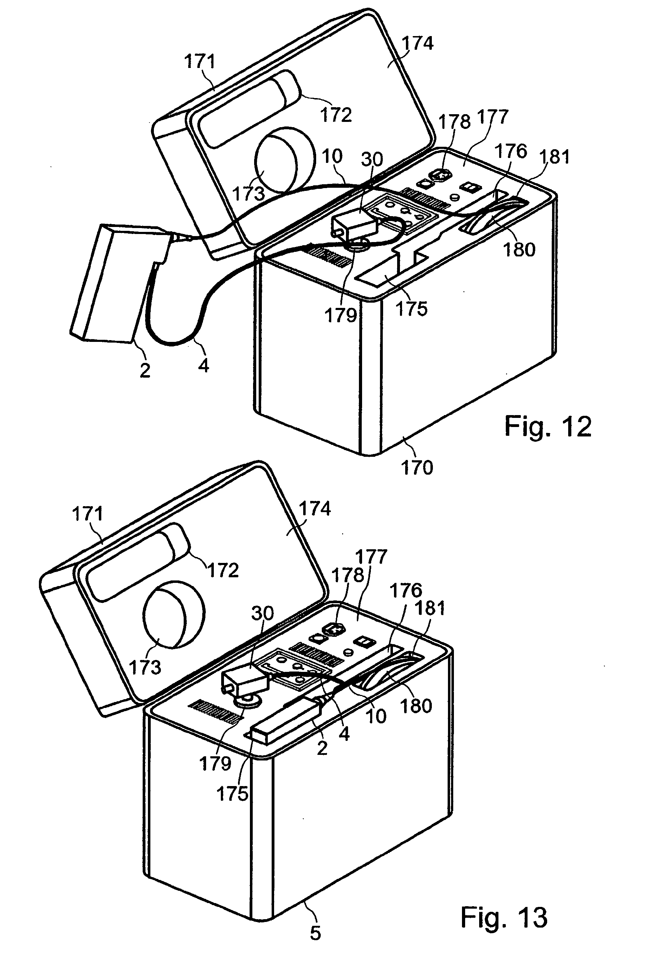

Les figures 12 et 13 représentent un coffret 170 de rangement et de transport de la sonde vidéoendoscopique décrite ci-avant. Le coffret 170 est muni d'un couvercle 171 amovible et renferme un dispositif d'alimentation électrique et un générateur d'éclairage. La face supérieure 177 du coffret 170 comprend notamment une embase de connexion 178 raccordable au secteur pour alimenter le dispositif d'alimentation électrique et le générateur d'éclairage, et une embase de connexion d'éclairage 179 dans laquelle est enfiché l'embout de connexion du boîtier de connexion 30 pour le raccordement du câble ombilical 4.

Le coffret 170 comprend également un logement capitonné 175 destiné à recevoir la poignée de commande 2, un logement capitonné 176 destiné à recevoir le câble ombilical 4, et une ouverture 181 d'accès à logement de rangement du tube d'inspection 10.

Comme cela est montré sur la figure 13, l'ensemble de la sonde comportant la poignée de commande 2, le tube d'inspection 10 et le câble ombilical 4 peut être logé dans le coffret de rangement 170 sans avoir à débrancher la sonde.

Le couvercle 171 dispose d'une plaque de mousse de protection 174 présentant deux cavités 172, 173 servant de logement et de protection, lorsque le couvercle est refermé sur le coffret, des parties de la poignée de commande 2 et du boîtier de connexion 30 qui dépassent de la face supérieure 177 du coffret 170.

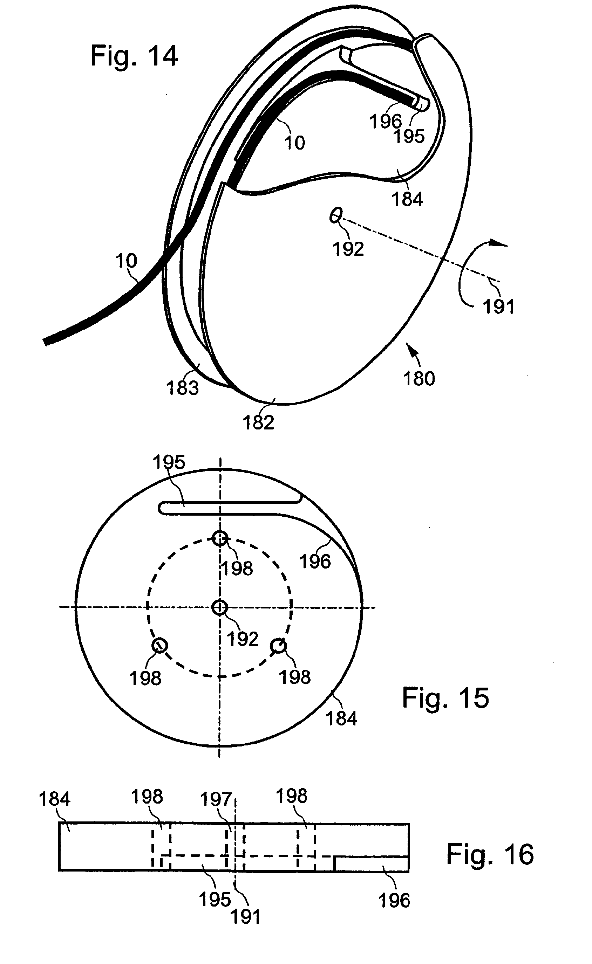

Selon l'invention, l'ouverture 181 pour le rangement du tube d'inspection 10 donne accès à un tambour 180 monté librement rotatif, une partie de la surface cylindrique du tambour étant visible par la fenêtre 181. Ce tambour est montré plus en détail sur la figure 14.
Sur cette figure, le tambour 180 comprend d'une manière classique deux flasques latérales 182, 183 en forme de disque, maintenant entre elles coaxialement un moyeu 184 cylindrique, dont le diamètre est légèrement inférieur à celui des flasques, le tambour étant mobile en rotation autour de son axe 191.

Lorsque le couvercle 171 est refermé sur le coffret 170, le tambour 180 est avantageusement immobilisé par la plaque de mousse 174 du fait que les flasques latérales du tambour 180 dépassent légèrement de la face supérieure 177 et donc pénètrent légèrement dans la plaque de mousse 174.

Tel que représenté sur les figures 14 à 16, le moyeu 184 comprend un alésage central 192 destiné à recevoir un axe de rotation et des perçages 198 de fixation des flaques latérales 182, 183. Selon l'invention, le moyeu 184 comprend également une cavité 195 destinée à recevoir l'embout distal 12 du tube d'inspection 10. Cette cavité présente une forme tubulaire et débouche tangentiellement à la surface cylindrique du moyeu 184 par une zone de transition 196 présentant une surface cylindrique de rayon inférieur à celui du moyeu.

Avantageusement, la cavité 195 est usinée sur l'une des faces latérales du moyeu 184 et est refermée latéralement par une des flasques 182, 183.

Pour ranger le tube d'inspection 10 dans le coffret 170, il suffit d'introduire l'embout distal 12 du tube d'inspection 10 dans la cavité 195 du tambour 180, qui est accessible par la fenêtre 181 du coffret 170, et de pousser sur le tube d'inspection. Comme la cavité 195 est tangente à la surface cylindrique du moyeu, la force d'insertion exercée sur le tube d'inspection 10 fait tourner le tambour 180. A partir d'une certaine longueur enroulée du tube d'inspection, on peut faire tourner le tambour à la main autour de l'axe 191, en retenant légèrement le tube d'inspection pour que ce dernier reste plaqué sur le tambour.

Grâce à ces dispositions, l'embout distal 12 du tube d'inspection est parfaitement protégé dans la cavité 195 et la force nécessaire pour entraîner le tambour en rotation est constante quelle que soit la longueur de tube déjà enroulée, contrairement au tube en spiral de l'art antérieur.

En outre, comme le tambour peut être facilement entraîné en rotation à la main par l'ouverture 181, il n'est pas nécessaire de prévoir des moyens d'entraînement du tambour.

Avantageusement, le moyeu 184 dans lequel est formée la cavité 195 est réalisé dans une matière présentant un coefficient de frottement élevé. De cette manière, l'embout distal 12 de la sonde est maintenu dans la cavité 195 avec une plus grande efficacité durant l'enroulement du tube d'inspection autour du moyeu. Ainsi, le moyeu est par exemple réalisé en une mousse alvéolaire dure qui présente l'avantage d'être légère, d'avoir un coefficient de frottement élevé et d'être facilement usinable.

De préférence, le diamètre intérieur de la cavité cylindrique 195 est légèrement supérieur au plus grand diamètre d'embout distal de tube d'inspection susceptible d'être enroulé autour du tambour 180.

## Revendications

1. Vidéoendoscope comprenant :
- une sonde d'inspection comprenant un tube d'inspection (10) comportant un embout distal (12) logeant un dispositif optoélectronique d'imagerie (14, 15, 16) délivrant un signal électrique,
- un processeur vidéo (39) pour traiter le signal électrique délivré par le dispositif d'imagerie afin de générer un signal vidéo,
- une poignée de commande comprenant un boîtier de commande (2) solidaire de l'extrémité proximale du tube d'inspection (10) et muni de moyens (23 à 26) de commande et de réglage du processeur vidéo (39),
- un câble ombilical (4) de raccordement dont l'extrémité distale est solidaire du boîtier de commande, pour connecter le vidéoendoscope à une source de lumière (52) et à une source d'alimentation électrique (51, 90),
- un boîtier de visualisation (3, 3', 3") intégrant un écran vidéo d'affichage (5) connecté au processeur vidéo pour visualiser le signal vidéo, et
- un faisceau de fibres d'éclairage (17) intégré sans rupture de continuité dans le câble ombilical, dans la poignée de commande, puis dans le tube d'inspection et dont l'extrémité distale, logée dans l'embout distal, illumine une cible observée par la sonde lorsque l'extrémité proximale du câble ombilical est connectée à un générateur de lumière,
dans lequel le boîtier de visualisation (3, 3', 3") est solidaire d'une face latérale (28) du boîtier de commande (2), le boîtier de commande comprenant sur une face supérieure un panneau de commande (20) muni des moyens (23 à 26) de commande et de réglage du processeur vidéo (39), et présentant une forme allongée entre son extrémité distale et son extrémité proximale, de manière à pouvoir être tenu dans une main et à permettre l'actionnement des organes de commande à l'aide du pouce de ladite main,
**caractérisé en ce que** le boîtier de visualisation (3') est solidarisé avec le boîtier de commande (2) par une articulation (9) permettant d'orienter l'écran d'affichage (5) autour d'un axe perpendiculaire à la face latérale (28) du boîtier de commande (2), le boîtier de visualisation (3') étant couplé électriquement au boîtier de commande (2) par l'intermédiaire de conducteurs électriques passant dans l'articulation (9).

2. Vidéoendoscope selon la revendication 1, **caractérisé en ce que** le câble ombilical (4) est raccordé au boîtier de commande (2) par une face latérale de celui-ci.

3. Vidéoendoscope selon la revendication 2, **caractérisé en ce que** l'articulation (9) est formée d'un connecteur assurant un raccordement mécanique et électrique amovible du boîtier de visualisation (3') au boîtier de commande (2).

4. Vidéoendoscope selon l'une des revendications 1 à 3, **caractérisé en ce que** l'extrémité proximale du câble ombilical (4) est solidaire d'un boîtier de connexion (30) équipé de moyens de connexion (34) de l'extrémité proximale (37) du faisceau de fibres d'éclairage (17) à un générateur de lumière (52), et de moyens de connexion (31, 44) de la sonde vidéoendoscopique à une source d'alimentation électrique (51, 90).

5. Vidéoendoscope selon la revendication 4, **caractérisé en ce que** l'embout de connexion (34) comprend des moyens de fixation (36) pour fixer un adaptateur mécanique (35) pour adapter l'embout de connexion à l'embout de connexion de n'importe quel générateur de lumière, le panneau de commande (20) comprend un organe de commande (26) pour commander l'initialisation du processeur vidéo (39) en fonction de la température de couleur de la lampe du générateur de lumière.

6. Vidéoendoscope selon l'une des revendications 1 à 5, **caractérisé en ce que** le processeur vidéo (39) est intégré dans le boîtier commande (2).

7. Vidéoendoscope selon la revendication 6, **caractérisé en ce que** le boîtier de commande (2) comprend sur ses deux faces latérales des moyens d'accouplement mécanique et électrique pour la fixation et la connexion électrique du boîtier de visualisation (3, 3', 3") indifféremment sur l'une ou l'autre des faces latérales du boîtier de commande.

8. Vidéoendoscope selon l'une des revendications 1 à 5, **caractérisé en ce que** le processeur vidéo (39) est intégré dans le boîtier de visualisation (3, 3').

9. Vidéoendoscope selon l'une des revendications 1 à 5, **caractérisé en ce que** le processeur vidéo (39) est intégré dans un boîtier de connexion (30) de l'extrémité proximale du câble ombilical (4) à un coffret externe.

10. Vidéoendoscope selon l'une des revendications 4 à 9, **caractérisé en ce que** le boîtier de connexion (30) comprend en outre des moyens de connexion (33) de la sonde à un système (58) de traitement et/ou de stockage d'images, la sonde vidéoendoscopique comprenant en outre des moyens de commutation (80) conçus pour orienter vers l'écran vidéo (5) soit le signal vidéo issu du processeur vidéo (39), soit le signal vidéo issu du système (58) de traitement et/ou de stockage d'images.

11. Vidéoendoscope selon la revendication 10, **caractérisé en ce que** le boîtier de commande (2) comprend des moyens (68, 20) pour commander un système (58) de traitement et/ou de stockage d'images connecté aux moyens de connexion (33) du dispositif de connexion (30), ces moyens de connexion comprenant une broche (46) pour transmettre le signal vidéo généré par le processeur vidéo (39) à une entrée vidéo (56) du système de traitement et/ou de stockage d'images, une broche (43) pour transmettre au dispositif de commutation (80) un signal vidéo généré par le système de traitement et/ou de stockage d'images, et une broche (45) reliant les moyens de commande (68, 20) du boîtier de commande (2) à une interface de commande du système de traitement et/ou de stockage d'images.

12. Vidéoendoscope selon la revendication 10, **caractérisé en ce qu'**il comprend en outre des moyens pour commander les moyens de commutation (80) pour orienter automatiquement vers l'écran vidéo (5) le signal vidéo issu du système (58) de traitement et/ou de stockage d'images dès que celui-ci est raccordé aux moyens de connexion (33).

13. Vidéoendoscope selon la revendication 10, **caractérisé en ce que** les moyens de commutation (80) sont intégrés dans le boîtier de commande (2).

14. Vidéoendoscope selon la revendication 10, **caractérisé en ce que** les moyens de commutation (80) sont intégrés dans le boîtier de visualisation (3).

15. Vidéoendoscope selon la revendication 10, **caractérisé en ce que** les moyens de commutation (80) sont intégrés dans le dispositif de connexion (30).

16. Vidéoendoscope selon l'une des revendications 10 à 15, **caractérisé en ce que** les moyens de connexion (33) de la sonde à un système (58) de traitement et/ou de stockage d'images comprennent une broche (44) de connexion permettant d'alimenter le vidéoendoscope à partir d'une alimentation électrique (51) associée au système (58) de traitement et/ou de stockage d'images.

17. Vidéoendoscope selon l'une des revendications 10 à 15, **caractérisé en ce que** le dispositif de connexion (30) comprend des moyens de connexion (31) de la sonde vidéoendoscopique à une source d'alimentation électrique auxiliaire (90).

18. Vidéoendoscope selon l'une des revendications 10 à 17, **caractérisé en ce que** le dispositif de connexion (30) comprend des moyens de connexion (32) de la sonde vidéoendoscopique à un moniteur vidéo auxiliaire (93), les moyens de commutation (80) comportant des moyens pour envoyer le signal vidéo appliqué en entrée de l'écran vidéo (5), vers les moyens de connexion (32) d'un moniteur vidéo auxiliaire.

19. Vidéoendoscope selon l'une des revendications 10 à 18, **caractérisé en ce que** la poignée de commande (2) comprend des moyens (68, 20) pour commander les moyens de commutation (80).

20. Vidéoendoscope selon l'une des revendications 1 à 19, **caractérisé en ce que** le boîtier de commande (2) intègre en outre :
- un dispositif électromécanique (27) conçu pour déformer un béquillage distal (11) déformable, intégré à l'extrémité distale (12) du tube d'inspection (10), afin de modifier l'orientation de l'extrémité distale du tube d'inspection et donc de la fenêtre d'observation de la sonde, le dispositif électromécanique (27) comprenant deux moteurs (114, 115) actionnant le béquillage distal par l'intermédiaire de deux paires respectives de câbles (104, 106 ; 103, 105), pour modifier l'orientation suivant deux plans respectifs, de l'extrémité distale du tube d'inspection,
- un processeur (151) délivrant deux signaux de commande (146, 147) appliqués respectivement aux deux moteurs, et
- des moyens d'introduction de commandes (21) comportant deux organes d'introduction de commandes, connectés au processeur pour introduire des commandes destinées respectivement aux deux moteurs, chacun des deux organes de commande présentant un premier état dans lequel le processeur commande le moteur respectif de manière à maintenir fixe l'orientation de l'extrémité distale du tube d'inspection, un second et un troisième états dans lesquels le processeur commande le moteur respectif de manière à faire varier, respectivement dans un sens et dans un sens opposé, l'orientation de l'extrémité distale du tube d'inspection.

21. Vidéoendoscope selon la revendication 20, **caractérisé en ce que**
chacun des moteurs (114, 115) est de type servomoteur actionnant une poulie (118, 119) couplée à une paire de câbles respective (104, 106 ; 103, 105), et dont la position angulaire est commandée par le signal de commande (146, 147) respectif généré par le processeur (151) et appliqué au moteur, chaque signal de commande présentant la forme d'un train d'impulsions, la largeur des impulsions correspondant à une position angulaire déterminée de la poulie, le processeur (151) comprenant des moyens pour maintenir constante la largeur des impulsions de chaque signal de commande lorsque l'organe de commande respectif se trouve dans le premier état, et augmentant et diminuant la largeur des impulsions à une vitesse prédéfinie lorsque l'organe de commande respectif se trouve respectivement dans les second et troisième états.

22. Vidéoendoscope selon la revendication 20 ou 21, **caractérisé en ce qu'**il comprend un organe de commande supplémentaire (22) intégré dans le panneau de commande (20) et connecté au processeur (151) pour commander la largeur des impulsions des signaux de commande appliqués aux moteurs (114, 115), de manière à ce qu'elle soit égale à une valeur médiane correspondant à une déformation nulle du béquillage distal (11).

23. Vidéoendoscope selon la revendication 22, **caractérisé en ce que** l'organe de commande supplémentaire (22) est intégré aux moyens d'introduction de commandes (21) du béquillage distal (11).

24. Vidéoendoscope selon l'une des revendications 20 à 23, **caractérisé en ce que** les deux organes de commande comprennent chacun une paire de contacts (141, 143 ; 142, 144) qui sont ouverts dans le premier état, l'un ou l'autre des contacts étant fermé dans les second et troisième états.

25. Vidéoendoscope selon la revendication 24, **caractérisé en ce que** chacun des organes de commande comprend deux boutons poussoirs intégrés dans le panneau de commande (20), pour actionner respectivement les deux contacts (141, 143 ; 142, 144) qui sont à l'état ouverts au repos, et passent à l'état fermé pendant que le bouton poussoir respectif est maintenu enfoncé.

26. Vidéoendoscope selon l'une des revendications 20 à 24, **caractérisé en ce que** les moyens d'introduction de commandes du béquillage comprennent un manche à balai (21) capable d'actionner simultanément les deux organes d'introduction de commande.

27. Vidéoendoscope selon l'une des revendications 20 à 26, **caractérisé en ce qu'**il comprend un organe de commande (149) supplémentaire intégré au panneau de commande (20) pour modifier la vitesse de variation de l'orientation de l'extrémité distale du tube d'inspection (10) en sélectionnant une vitesse lente ou une vitesse rapide.

28. Vidéoendoscope selon l'une des revendications 20 à 26, **caractérisé en ce que** le processeur (151) est programmé pour sélectionner une vitesse rapide de variation de l'orientation de l'extrémité distale du tube d'inspection (10) si au moins un des deux organes de commande est maintenu dans le second ou troisième état pendant une durée supérieure à un seuil prédéfini, et pour sélectionner une vitesse lente de variation de l'orientation de l'extrémité distale du tube d'inspection si les deux organes de commande sont dans le premier état.

29. Vidéoendoscope selon l'une des revendications 20 à 28, **caractérisé en ce que** le processeur (151) est programmé pour déterminer l'orientation de l'extrémité distale (12) du tube d'inspection (10) en fonction de forme des signaux de commande appliqués respectivement aux deux moteurs (114, 115), et pour afficher à l'écran d'affichage (5) des symboles représentant l'orientation déterminée.

30. Vidéoendoscope selon l'une des revendications 1 à 29, **caractérisé en ce** qu"il comprend un coffret (170) de rangement et de transport renfermant une alimentation électrique (51) et un générateur d'éclairage (52), le coffret de rangement comprenant un tambour (180) autour duquel la sonde (10) peut être enroulée, le tambour étant monté de manière à pouvoir tourner librement autour de son axe (191) et comprenant une cavité tubulaire (195) destinée à recevoir l'extrémité distale (12) de la sonde (10), et débouchant tangentiellement à la surface cylindrique du tambour (180).

31. Vidéoendoscope selon la revendication 30, **caractérisé en ce que** le tambour (180) comprend un moyeu central (184) maintenu entre deux flasques coaxiales (182, 183), la cavité (195) étant réalisée à partir d'une des faces latérales du moyeu, et refermée latéralement par l'un des deux flasques.

32. Vidéoendoscope selon la revendication 30, **caractérisé en ce que** le moyeu central (184) est réalisé dans une matière présentant un coefficient de frottement élevé.

33. Vidéoendoscope selon la revendication 31, **caractérisé en ce que** le moyeu central (184) est réalisé dans une mousse alvéolaire dure.

34. Vidéoendoscope selon l'une des revendications 30 à 32, **caractérisé en ce que** le coffret de rangement (170) comprend un logement dans lequel est fixé le tambour (180), le logement présentant une ouverture (181) donnant accès à une partie de la surface cylindrique du tambour.

35. Videoendoscope selon l'une des revendications 30 à 33, **caractérisé en ce que** le coffret (170) comprend un couvercle (171) dont la face interne est recouverte de mousse (174), le tambour (180) étant monté dans le coffret de manière à être bloqué en rotation par la mousse du couvercle lorsque ce dernier est refermé sur le coffret.

36. Vidéoendoscope selon l'une des revendications 30 à 34, **caractérisé en ce que** le diamètre intérieur de la cavité cylindrique (195) est légèrement supérieur au plus grand diamètre de l'extrémité distale (12) de la sonde (10) susceptible d'être enroulée autour du tambour (180).

## Claims

1. A videoendoscope comprising:
- an inspection probe comprising an inspection tube (10) comprising a distal tip (12) housing an imagery optoelectronic device (14, 15, 16) supplying an electrical signal,
- a video processor (39) for processing the electrical signal supplied by the imagery device so as to generate a video signal,
- a control handle comprising a control box (2) attached to the proximal end of the inspection tube (10) and provided with means (23 to 26) for controlling and setting the video processor (39),
- a connection umbilical cable (4) which distal end is attached to the control box, for connecting the videoendoscope to a source of light (52) and a power supply source (51, 90),
- a visualization box (3, 3', 3") integrating a display video screen (5) connected to the video processor for visualizing the video signal, and
a beam of lighting fibres (17) integrated without continuity break into the umbilical cable, the control handle, and in the inspection tube and which distal end, housed in the distal tip, lights a target observed by the probe when the proximal end of the umbilical cable is connected to a light generator,
wherein the visualization box (3, 3', 3") is attached to a lateral face (28) of the control box (2), the control box comprising on an upper face a control panel (20) provided with the means (23 to 26) for controlling and setting the video processor (39), and having an elongated shape between the distal end and the proximal end thereof, so as to be able to be held in one hand and to allow the control elements to be operated using the thumb of the hand,
**characterised in that** the visualization box (3') is attached to the control box (2) through a joint (9) allowing the display screen (5) to be oriented around an axis perpendicular to the lateral face (28) of the control box (2), the visualization box (3') being electrically coupled to the control box (2) through electrical conductors passing through the joint (9).

2. Videoendoscope according to claim 1,
**characterised in that** the umbilical cable (4) is connected to the control box (2) by a lateral face thereof.

3. Videoendoscope according to claim 2,
**characterised in that** the joint (9) is formed by a connector performing a removable mechanical and electrical connection of the visualisation box (3') to the control box (2).

4. Videoendoscope according to one of claims 1 to 3, **characterised in that** the proximal end of the umbilical cable (4) is attached to a connection box (30) provided with connection means (34) for connecting the proximal end (37) of the beam of lighting fibres (17) to a light generator (52), and connection means (31, 44) for connecting the videoendoscopic probe to a power supply source (51, 90).

5. Videoendoscope according to claim 4,
**characterised in that** the connection tip (34) comprises fixation means (36) for fixing a mechanical adapter (35) for adapting the connection tip to the connection tip of any light generator, the control panel (20) comprises a control element (26) for controlling the initialisation of the video processor (39) according to the colour temperature of the lamp of the light generator.

6. Videoendoscope according to one of claims 1 to 5, **characterised in that** the video processor (39) is integrated into the control box (2).

7. Videoendoscope according to claim 6,
**characterised in that** control box (2) comprises on its two lateral faces mechanical and electrical coupling means for fixing and electrically connecting the visualisation box (3, 3', 3") equally on one or the other lateral face of the control box.

8. Videoendoscope according to one of claims 1 to 5, **characterised in that** the video processor (39) is integrated into the visualization box (3, 3').

9. Videoendoscope according to one of claims 1 to 5, **characterised in that** the video processor (39) is integrated into a connection box (30) for connecting the proximal end of the umbilical cable (4) to an external box.

10. Videoendoscope according to one of claims 4 to 9, **characterised in that** the connection box (30) also comprises connection means (33) for connecting the probe to a system (58) for processing and/or storing images, the videoendoscopic probe also comprising switching means (80) designed to direct toward the video screen (5) either the video signal coming from the video processor (39), or the video signal coming from the system (58) for processing and/or storing images.

11. Videoendoscope according to claim 10,
**characterised in that** the control box (2) comprises means (68, 20) for controlling a system (58) for processing and/or storing images connected to the connection means (33) of the connection device (30), the connection means comprising a pin (46) for transmitting the video signal generated by the video processor (39) to a video input (56) of the system for processing and/or storing images, a pin (43) for transmitting to the switching device (80) a video signal generated by the system for processing and/or storing images, and a pin (45) linking the control means (68, 20) of the control box (2) to a control interface for controlling the system for processing and/or storing images.

12. Videoendoscope according to claim 10,
**characterised in that** it also comprises means for controlling the switching means (80) to automatically direct toward the video screen (5) the video signal coming from the system (58) for processing and/or storing images as soon as it is connected to the connection means (33).

13. Videoendoscope according to claim 10,
**characterised in that** the switching means (80) are integrated into the control box (2).

14. Videoendoscope according to claim 10,
**characterised in that** the switching means (80) are integrated into the visualisation box (3).

15. Videoendoscope according to claim 10,
**characterised in that** the switching means (80) are integrated into the connection device (30).

16. Videoendoscope according to one of claims 10 to 15, **characterised in that** the connection means (33) for connecting the probe to a system (58) for processing and/or storing images comprise a connection pin (44) allowing the videoendoscope to be powered from an electrical power supply (51) associated to the system (58) for processing and/or storing images.

17. Videoendoscope according to one of claims 10 to 15, **characterised in that** the connection device (30) comprises connection means (31) for connecting the videoendoscopic probe to an auxiliary electrical power supply (90).

18. Videoendoscope according to one of claims 10 to 17, **characterised in that** the connection device (30) comprises connection means (32) for connecting the videoendoscopic probe to an auxiliary video monitor (93), the switching means (80) comprising means for sending the video signal applied to the input of the video screen (5), toward the connection means (32) of an auxiliary video monitor.

19. Videoendoscope according to one of claims 10 to 18, **characterised in that** the control handle (2) comprises means (68, 20) for controlling the switching means (80).

20. Videoendoscope according to one of claims 1 to 19, **characterised in that** the control box (2) also integrates:
- an electromechanical device (27) designed to distort a deformable distal deflection tip (11), integrated into the distal end (12) of the inspection tube (10), so as to modify the orientation of the distal end of the inspection tube and therefore of the observation window of the probe, the electromechanical device (27) comprising two motors (114, 115) operating the distal deflection tip through two respective couples of cables (104, 106; 103, 105), for modifying the orientation according to two respective planes, of the distal end of the inspection tube,
- a processor (151) supplying two control signals (146, 147) respectively applied to the two motors, and
- means for inputting commands (21) comprising two elements for inputting commands, connected to the processor for inputting commands respectively intended for the two motors, each control element having a first state in which the processor controls the respective motor so as to keep the orientation of the distal end of the inspection tube fixed, and a second and a third states in which the processor controls the respective motor so as to modify, respectively in one direction and an opposite direction, the orientation of the distal end of the inspection tube.

21. Videoendoscope according to claim 20,
**characterised in that** each motor (114, 115) is of the servomotor type operating a pulley (118, 119) coupled to a respective couple of cables (104, 106; 103, 105), and which angular position is controlled by the respective control signal (146, 147) generated by the processor (151) and applied to the motor, each control signal having the shape of a pulse train, the width of pulses corresponding a determined angular position of the pulley, the processor (151) comprising means for keeping the width of the pulses of each control signal constant when the respective control element is in the first state, and increasing and decreasing the width of the pulses at a predefined rate when the respective control element is respectively in the second and third states.

22. Videoendoscope according to claim 20 or 21, **characterised in that** it comprises an additional control element (22) integrated into the control panel (20) and connected to the processor (151) for controlling the width of the pulses of the control signals applied to the motors (114, 115), so that it is equal to a median value corresponding a null distortion of the distal deflection tip (11).

23. Videoendoscope according to claim 22,
**characterised in that** the additional control element (22) is integrated to the command inputting means (21) of the distal deflection tip (11).

24. Videoendoscope according to one of claims 20 to 23, **characterised in that** the two control elements comprise each a couple of contacts (141, 143; 142, 144) which are open in the first state, one or the other of the contacts being closed in the second and third states.

25. Videoendoscope according to claim 24,
**characterised in that** each control element comprises two push buttons integrated into the control panel (20), for respectively operating the two contacts (141, 143; 142, 144) which are in the open state when idle, and switch to the closed state when the respective push button is maintained in the pushed position.

26. Videoendoscope according to one of claims 20 to 24, **characterised in that** the command inputting means of the deflection tip comprise a joystick (21) able to simultaneously operate the two command inputting elements.

27. Videoendoscope according to one of claims 20 to 26, **characterised in that** it comprises an additional control element (149) integrated into the control panel (20) for modifying the variation rate of the orientation of the distal end of the inspection tube (10) by choosing a slow rate or a rapid rate.

28. Videoendoscope according to one of claims 20 to 26, **characterised in that** the processor (151) is programmed to select a rapid variation rate of the orientation of the distal end of the inspection tube (10) if at least one of the two control elements is maintained in the second or third state during a duration higher than a predefined threshold, and to select a slow variation rate of the orientation of the distal end of the inspection tube if the two control elements are in the first state.

29. Videoendoscope according to one of claims 20 to 28, **characterised in that** the processor (151) is programmed to determine the orientation of the distal end (12) of the inspection tube (10) according to the shape of the control signals respectively applied to the two motors (114, 115), and to display on the display screen (5) symbols representing the orientation determined.

30. Videoendoscope according to one of claims 1 to 29, **characterised in that** it comprises a storage and transport box (170) comprising an electrical power supply (51) and a light generator (52), the storage box comprising a drum (180) around which the probe (10) may be winded, the drum being mounted so as to be able to freely turn around its axis (191) and comprising a tubular cavity (195) intended to receive the distal end (12) of the probe (10), and tangentially opening onto the cylindrical surface of the drum (180).

31. Videoendoscope according to claim 30,
**characterised in that** the drum (180) comprises a central hub (184) maintained between two coaxial flanges (182, 183), the cavity (195) being made from one of the lateral faces of the hub, and being laterally closed by one of the two flanges.

32. Videoendoscope according to claim 30,
**characterised in that** the central hub (184) is made in a material having a high coefficient of friction.

33. Videoendoscope according to claim 31,
**characterised in that** the central hub (184) is made in a hard cellular foam.

34. Videoendoscope according to one of claims 30 to 32, **characterised in that** the storage box (170) comprises a housing in which the drum (180) is fixed, the housing having an opening (181) providing access to a part of the cylindrical surface of the drum.

35. Videoendoscope according to one of claims 30 to 33, **characterised in that** the box (170) comprises a cover (171) which internal face is covered with foam (174), the drum (180) being mounted in the box so as to be blocked in rotation by the foam of the cover when it is closed onto the box.

36. Videoendoscope according to one of claims 30 to 34, **characterised in that** the internal diameter of the cylindrical cavity (195) is slightly higher than the greater diameter of the distal end (12) of the probe (10) susceptible of being winded around the drum (180).

## Patentansprüche

1. Videoendoskop umfassend
- eine Inspektionssonde, umfassend einen Inspektionsschlauch (10) mit einem distalen Ansatzstück (12), in welchem eine ein elektrisches Signal abgebende optoelektronische Bildgebungsvorrichtung (14, 15, 16) aufgenommen ist,
- einen Videoprozessor (39) für die Verarbeitung des von der Bildgebungsvorrichtung abgegebenen elektrischen Signals, um ein Videosignal zu generieren,
- einen Bedienungshebel, der eine mit dem Proximalende des Inspektionsschlauchs (10) fest verbundene und mit Mitteln (23 bis 26) zur Steuerung und Einstellung des Videoprozessors (39) versehene Steuerbox (2) umfasst,
- ein Nabelschnur-Anschlusskabel (4), dessen Distalende fest mit der Steuerbox verbunden ist, um das Videoendoskop an eine Lichtquelle (52), sowie an eine Stromspeisequelle (51, 90) anzuschließen,
- eine Visualisierungsbox (3, 3', 3 "), in die zur Anzeige des Videosignals ein an den Videoprozessor angeschlossener Video-Anzeigebildschirm (5) integriert ist, und
- ein Beleuchtungsfaserbündel (17), das ohne Kontinuitätsverlust in das Nabelschnurkabel, in den Bedienungshebel und dann in den Inspektionsschlauch integriert ist, und dessen im distalen Ansatzstück untergebrachtes Distalende ein von der Sonde beobachtetes Ziel beleuchtet, wenn das proximale Ende des Nabelschnurkabels an einen Lichtgenerator angeschlossen ist,
wobei die Visualisierungsbox (3, 3', 3") mit einer Seitenwand (28) der Steuerbox (2) fest verbunden ist, welche auf einer Oberseite ein Bedienungsfeld (20) umfasst, das mit Mitteln (23 bis 26) zur Steuerung und Einstellung des Videoprozessors (39) ausgestattet ist und zwischen seinem Distal- und seinem Proximalende eine längliche Form aufweist, so dass sie in einer Hand gehalten und die Steuerorgane mit Hilfe des Daumens dergenannten Hand betätigt werden können,
**dadurch gekennzeichnet, dass** die Visualisierungsbox (3') über ein Gelenk (9) fest mit der Steuerbox (2) verbunden ist, so dass die Sichtanzeige (5) um eine zur Seitenwand (28) der Steuerbox (2) senkrechte Achse ausgerichtet werden kann, wobei die Visualisierungsbox (3') über im Gelenk (9) verlaufende elektrische Leiter mit der Steuerbox (2) elektrisch gekoppelt ist.

2. Videoendoskop nach Anspruch 1, **dadurch gekennzeichnet, dass** das Nabelschnurkabel (4) über eine Seitenwand der Steuerbox (2) an diese angeschlossen ist.

3. Videoendoskop nach Anspruch 2, **dadurch gekennzeichnet, dass** das Gelenk (9) von einem Verbinder gebildet wird, über den ein lösbarer mechanischer und elektrischer Anschluss der Visualisierungsbox (3') an die Steuerbox (2) erfolgt.

4. Videoendoskop nach einem der Ansprüche 1 bis 3, **dadurch gekennzeichnet, dass** das Proximalende des Nabelschnurkabels (4) mit einer mit Mitteln (34) zum Anschluss des Proximalendes (37) des Leuchtfaserbündels (17) an einen Lichtgenerator (52), sowie mit Mitteln (31, 44) zum Anschluss der videoendoskopischen Sonde an eine Stromspeisequelle (51, 90) versehenen Verbindungsbox (30) fest verbunden ist.

5. Videoendoskop nach Anspruch 4, **dadurch gekennzeichnet, dass** das Anschlussstück (34) Befestigungsmittel (36) zur Befestigung eines mechanischen Adapters (35) umfasst, um das Anschlussstück an das Anschlussstück eines beliebigen Lichtgenerators anzupassen, wobei das Bedienungsfeld (20) ein Steuerorgan (26) umfasst, um die Initialisierung des Videoprozessors (39) in Abhängigkeit von der Farbtemperatur der Lampe des Lichtgenerators zu steuern.

6. Videoendoskop nach einem der Ansprüche 1 bis 5, **dadurch gekennzeichnet, dass** der Videoprozessor (39) in die Steuerbox (2) integriert ist.

7. Videoendoskop nach Anspruch 6, **dadurch gekennzeichnet, dass** die Steuerbox (2) auf ihren beiden Seitenwänden mechanische und elektrische Kopplungsmittel umfasst, um die Visualisierungsbox (3, 3', 3") unterschiedslos an der einen oder anderen Seitenwand der Steuerbox zu befestigen und elektrisch anzuschließen.

8. Videoendoskop nach einem der Ansprüche 1 bis 5, **dadurch gekennzeichnet, dass** der Videoprozessor (39) in die Visualisierungsbox (3, 3') integriert ist.

9. Videoendoskop nach einem der Ansprüche 1 bis 5, **dadurch gekennzeichnet, dass** der Videoprozessor (39) in eine Box (30) zur Verbindung des Proximalendes des Nabelschnurkabels (4) an eine externe Einheit integriert ist.

10. Videoendoskop nach einem der Ansprüche 4 bis 9, **dadurch gekennzeichnet, dass** die Verbindungsbox (30) zudem Mittel (33) zur Verbindung der Sonde mit einem Bildverarbeitungs- und/oder -speichersystem (58) umfasst, wobei die videoendoskopische Sonde zudem Umschaltungsmittel (80) umfasst, die dazu ausgelegt sind, entweder das vom Videoprozessor (39) stammende Videosignal oder das vom Bildverarbeitungs- und/oder - speichersystem (58) stammende Videosignal auf den Videobildschirm auszurichten.

11. Videoendoskop nach Anspruch 10, **dadurch gekennzeichnet, dass** die Steuerbox (2) Mittel (68, 20) zur Steuerung eines an die Verbindungsmittel (33) der Verbindungsvorrichtung (30) angeschlossenen Bildverarbeitungs- und/oder -speichersystem (58) umfasst, wobei diese Verbindungsmittel einen Kontaktstift (46) zur Übertragung des vom Videoprozessor (39) generierten Videosignals an einen Videoeingang (56) des Bildverarbeitungs- und/oder -speichersystems umfasst, sowie einen Kontaktstift (43) zur Übertragung eines vom Bildverarbeitungs- und/oder -speichersystem generierten Videosignals an die Umschaltungsvorrichtung (80) und einen Kontaktstift (45), der die Steuermittel (68, 20) der Steuerbox (2) mit einer Steuerschnittstelle des Bildverarbeitungs- und/oder -speichersystems verbindet.

12. Videoendoskop nach Anspruch 10, **dadurch gekennzeichnet, dass** es zudem Mittel zur Steuerung der Umschaltungsmittel (80) umfasst, um das vom Bildverarbeitungs- und/oder -speichersystem (58) stammende Videosignal automatisch auf den Videobildschirm (5) auszurichten, sobald das Bildverarbeitungs- und/oder -speichersystem (58) an die Verbindungsmittel (33) angeschlossen ist.

13. Videoendoskop nach Anspruch 10, **dadurch gekennzeichnet, dass** die Umschaltungsmittel (80) in die Steuerbox (2) integriert sind.

14. Videoendoskop nach Anspruch 10, **dadurch gekennzeichnet, dass** die Umschaltungsmittel (80) in die Visualisierungsbox (3) integriert sind.

15. Videoendoskop nach Anspruch 10, **dadurch gekennzeichnet, dass** die Umschaltungsmittel (80) in die Verbindungsvorrichtung (30) integriert sind.

16. Videoendoskop nach einem der Ansprüche 10 bis 15, **dadurch gekennzeichnet, dass** die Mittel (33) zur Verbindung der Sonde mit einem Bildverarbeitungs- und/oder - speichersystem (58) einen Verbindungsstecker (44) umfassen, der die Speisung des Videoendoskops ausgehend von einem dem Bildverarbeitungs- und/oder -speichersystem (58) zugeordneten Netzteil (51) ermöglicht.

17. Videoendoskop nach einem der Ansprüche 10 bis 15, **dadurch gekennzeichnet, dass** die Verbindungsvorrichtung (30) Mittel (31) zur Verbindung der videoendoskopischen Sonde mit einer ergänzenden Stromspeisequelle (90) umfasst.

18. Videoendoskop nach einem der Ansprüche 10 bis 17, **dadurch gekennzeichnet, dass** die Verbindungsvorrichtung (30) Mittel (32) zur Verbindung der videoendoskopischen Sonde mit einem ergänzenden Videomonitor (93) umfasst, wobei die Umschaltungsmittel (80) Mittel umfassen, um das an den Eingang des Videobildschirms (5) angelegte Videosignal an die Verbindungsmittel (32) eines ergänzenden Videomonitors zu senden.

19. Videoendoskop nach einem der Ansprüche 1 bis 18, **dadurch gekennzeichnet, dass** der Bedienungshebel (2) Mittel (68, 20) zur Steuerung der Umschaltungsmittel (80) umfasst.

20. Videoendoskop nach einem der Ansprüche 1 bis 19, **dadurch gekennzeichnet, dass** zudem folgendes in die Steuerbox (2) integriert ist:
- eine elektromechanische Vorrichtung (27), die dazu ausgelegt ist, ein im Distalende (12) des Inspektionsschlauches (10) integriertes verformbares Distalablenkungssystem zu deformieren, um die Ausrichtung des Distalendes des Inspektionsschlauchs und damit des Beobachtungsfensters der Sonde zu verändern, wobei die elektromechanische Vorrichtung (27) zwei Motoren (114, 115) umfasst, die das Distalablenkungssystem über jeweils zwei Kabelpaare (104, 106 ; 103, 105) betätigt, um die Ausrichtung des Distalendes des Inspektionsschlauchs in jeweils zwei Ebenen zu ändern,
- einen Prozessor (151), der zwei jeweils an die beiden Motoren angelegte Steuersignale (146, 147) abgibt, und
- Mittel (21) zum Einleiten von Steuerbefehlen, die zwei mit dem Prozessor verbundene Befehlseinleitungsorgane umfassen, um Steuerbefehle einzuleiten, die jeweils für die beiden Motoren bestimmt sind, wobei jedes der beiden Steuerorgane einen ersten Zustand aufweist, in dem der Prozessor den jeweiligen Motor so steuert, dass die Ausrichtung des Distalendes des Inspektionsschlauchs stationär gehalten wird, sowie einen zweiten und einen dritten Zustand, in denen der Prozessor den jeweiligen Motor so steuert, dass die Ausrichtung des Distalendes des Inspektionsschlauchs jeweils in eine Richtung und in eine gegenüberliegende Richtung geändert wird.

21. Videoendoskop nach Anspruch 20, **dadurch gekennzeichnet, dass** jeder der Motoren (114, 115) von der Art eines Servomotors ist, der eine mit jeweils einem Kabelpaar (104, 106 ; 103, 105) gekoppelte Antriebsscheibe (118, 119) betätigt und dessen Winkelposition durch das jeweilige vom Prozessor (151) generierte Steuersignal (146, 147) gesteuert und an den Motor angelegt wird, wobei jedes Steuersignal die Form einer Impulsreihe aufweist, die Impulsbreite einer bestimmten Winkelposition der Antriebsscheibe entspricht und der Prozessor (151) Mittel umfasst, um die Impulsbreite jedes Steuersignals konstant zu halten, wenn sich das jeweilige Steuerorgan im ersten Zustand befindet und die Impulsbreite mit einer vorbestimmten Geschwindigkeit zu erhöhen und zu reduzieren, wenn sich das jeweilige Steuerorgan im zweiten oder dritten Zustand befindet.

22. Videoendoskop nach Anspruch 20 oder 21, **dadurch gekennzeichnet, dass** es ein zusätzliches, in die Bedienungsfeld (20) integriertes und an den Prozessor (151) angeschlossenes Steuerorgan (22) umfasst, um die Impulsbreite der an die Motoren (114, 115) angelegten Steuersignale so zu steuern, dass sie gleich einem Medianwert ist, der einer Null-Deformation des Distalablenkungssystems (11) entspricht.

23. Videoendoskop nach Anspruch 22, **dadurch gekennzeichnet, dass** das zusätzliche Steuerorgan (22) in die Befehlseinleitungsmittel (21) des Distalablenkungssystems (11) integriert ist.

24. Videoendoskop nach einem der Ansprüche 20 bis 23, **dadurch gekennzeichnet, dass** jedes der beiden Steuerorgane ein Kontaktpaar (141, 143 ; 142, 144) umfasst, das im ersten Zustand offen ist, wobei der eine oder andere der Kontakte im zweiten und dritten Zustand geschlossen ist.

25. Videoendoskop nach Anspruch 24, **dadurch gekennzeichnet, dass** jedes der Steuerorgane zwei in die Bedienungsfeld (20) integrierte Druckknöpfe umfasst, um jeweils die beiden Kontakte (141, 143 ; 142, 144) zu betätigen, die sich in der Ruhestellung im offenen Zustand befinden und die in den geschlossenen Zustand übergehen, während der jeweilige Druckknopf in gedrückter Stellung gehalten wird.

26. Videoendoskop nach einem der Ansprüche 20 bis 24, **dadurch gekennzeichnet, dass** die Befehlseinleitungsmittel des Ablenkungssystems einen Steuerhebel (21) umfassen, der in der Lage ist, die beiden Befehlseinleitungsorgane gleichzeitig zu betätigen.

27. Videoendoskop nach einem der Ansprüche 20 bis 26, **dadurch gekennzeichnet, dass** es ein zusätzliches, in die Bedienungsfeld (20) integriertes Steuerorgan umfasst, um die Veränderungsgeschwindigkeit der Ausrichtung des Distalendes des Inspektionsschlauchs (10) zu ändern, indem eine langsame oder schnelle Geschwindigkeit selektiert wird.

28. Videoendoskop nach einem der Ansprüche 20 bis 26, **dadurch gekennzeichnet, dass** der Prozessor (151) programmiert ist, um eine schnelle Veränderungsgeschwindigkeit der Ausrichtung des Distalendes des Inspektionsschlauchs (10) zu selektieren, wenn wenigstens eines der beiden Steuerorgane während eines über einer vorbestimmten Schwelle liegenden Zeitraums im zweiten oder dritten Zustand gehalten wird, und um eine langsame Veränderungsgeschwindigkeit der Ausrichtung des Distalendes des Inspektionsschlauchs (10) zu selektieren, wenn sich die beiden Steuerorgane im ersten Zustand befinden.

29. Videoendoskop nach einem der Ansprüche 20 bis 28, **dadurch gekennzeichnet, dass** der Prozessor (151) programmiert ist, um die Ausrichtung des Distalendes (12) des Inspektionsschlauchs (10) in Abhängigkeit von der Form der jeweils an die beiden Motoren (114, 115) angelegten Steuersignale festzulegen und um Symbole auf der Sichtanzeige (5) anzuzeigen, die die festgelegte Ausrichtung darstellen.

30. Videoendoskop nach einem der Ansprüche 1 bis 29, **dadurch gekennzeichnet, dass** es einen Stau- und Transportkoffer (170) umfasst, der ein Netzteil (51) und einen Beleuchtungsgenerator (52) enthält, wobei der Staukoffer eine Trommel (180) umfasst, auf die die Sonde (10) aufgewickelt werden kann und die so montiert ist, dass sie sich ungehindert um ihre Achse (191) drehen kann, und die einen röhrenförmigen Hohlraum (195) umfasst, der zur Aufnahme des Distalendes (12) der Sonde (10) bestimmt ist und der tangential zur zylindrischen Oberfläche der Trommel (180) mündet.

31. Videoendoskop nach Anspruch 30, **dadurch gekennzeichnet, dass** die Trommel (180) eine zentrale Nabe (184) umfasst, die zwischen zwei koaxialen Flanschen (182, 183) gehalten ist, wobei der Hohlraum (195) ausgehend von einer der Nabenseitenwände gebildet und seitlich von einem der beiden Flansche wieder verschlossen wird.

32. Videoendoskop nach Anspruch 30, **dadurch gekennzeichnet, dass** die zentrale Nabe (184) aus einem Werkstoff mit einem hohen Reibbeiwert gefertigt ist.

33. Videoendoskop nach Anspruch 31, **dadurch gekennzeichnet, dass** die zentrale Nabe (184) aus einem harten Zellschaumstoff gefertigt ist.

34. Videoendoskop nach einem der Ansprüche 30 bis 32, **dadurch gekennzeichnet, dass** der Staukoffer (170) eine Aufnahme umfasst, in der die Trommel (180) befestigt ist,
wobei die Aufnahme eine Öffnung (181) aufweist, durch die Zugang zu einem Teil der zylindrischen Oberfläche der Trommel besteht.

35. Videoendoskop nach einem der Ansprüche 30 bis 33, **dadurch gekennzeichnet, dass** der Staukoffer (170) einen Deckel (171) umfasst, dessen Innenseite mit Schaumstoff (174) überzogen ist, wobei die Trommel (180) so in dem Koffer montiert ist, dass sie vom Schaumstoff des Deckels drehfest blockiert ist, wenn der Deckel den Koffer verschließt.

36. Videoendoskop nach einem der Ansprüche 30 bis 34, **dadurch gekennzeichnet, dass** der Innendurchmesser des zylindrischen Hohlraums (195) geringfügig größer ist, als der größte Durchmesser des Distalendes (12) der um die Trommel (180) wickelbaren Sonde (10).
